(19) Europäisches Patentamt / European Patent Office / Office européen des brevets

(11) **EP 2 596 351 B1**

(12) **FASCICULE DE BREVET EUROPEEN**

(45) Date de publication et mention de la délivrance du brevet:
**25.06.2014 Bulletin 2014/26**

(21) Numéro de dépôt: **11752276.3**

(22) Date de dépôt: **21.07.2011**

(51) Int Cl.:
**G01N 33/50** *(2006.01)*　　　**G01N 33/543** *(2006.01)*
**G01N 33/58** *(2006.01)*　　　**B01L 3/00** *(2006.01)*
**G01N 21/64** *(2006.01)*

(86) Numéro de dépôt international:
**PCT/FR2011/051773**

(87) Numéro de publication internationale:
**WO 2012/010811 (26.01.2012 Gazette 2012/04)**

(54) **PROCÉDÉ ET DISPOSITIF D'ANALYSE D'INTERACTIONS MOLÉCULAIRES, ET LEURS UTILISATIONS**

VERFAHREN UND VORRICHTUNG ZUR ANALYSE MOLEKULARER WECHSELWIRKUNGEN UND VERWENDUNGEN DAVON

METHOD AND DEVICE FOR ANALYSING MOLECULAR INTERACTIONS, AND USES THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.07.2010 FR 1055940**

(43) Date de publication de la demande:
**29.05.2013 Bulletin 2013/22**

(73) Titulaire: **Ecole Polytechnique / DGAR**
**91128 Palaiseau Cedex (FR)**

(72) Inventeurs:
• **TURKCAN, Silvan**
**Los Altos, CA 64022 (US)**
• **ALLAIN, Jean-Marc**
**92160 Antony (FR)**
• **ALEXANDROU, Antigoni**
**91120 Palaiseau (FR)**

(74) Mandataire: **Novagraaf Technologies**
**122, rue Edouard Vaillant**
**92593 Levallois-Perret Cedex (FR)**

(56) Documents cités:
**WO-A2-03/095973　　WO-A2-2007/092909**

• **MIALON G ET AL: "Luminescent oxide nanoparticles with enhanced optical properties", JOURNAL OF LUMINESCENCE, AMSTERDAM, NL, vol. 129, no. 12, 1 décembre 2009 (2009-12-01), pages 1706-1710, XP026755075, ISSN: 0022-2313, DOI: DOI:10.1016/J.JLUMIN.2009.01.039 [extrait le 2009-11-11]**
• **MASSON J -B ET AL: "Inferring maps of forces inside cell membrane microdomains", PHYSICAL REVIEW LETTERS AMERICAN PHYSICAL SOCIETY USA, vol. 102, no. 4, 30 janvier 2009 (2009-01-30), XP002613796, ISSN: 0031-9007**
• **BEAUREPAIRE E ET AL: "Functionalized luminescent oxide nanoparticles for sodium channel imaging at the single molecule level", PROCEEDINGS OF THE SPIE - THE INTERNATIONAL SOCIETY FOR OPTICAL ENGINEERING SPIE - USA, vol. 5705, no. 1, 28 avril 2005 (2005-04-28), pages 192-198, XP002659644, ISSN: 0277-786X**
• **SCHMIDT B J ET AL: "Catch strip assay for the relative assessment of two-dimensional protein association kinetics", ANALYTICAL CHEMISTRY, AMERICAN CHEMICAL SOCIETY, US, vol. 80, no. 4, 15 February 2008 (2008-02-15), pages 944-950, XP002613795, ISSN: 0003-2700, DOI: 10.1021/AC071529I [retrieved on 2008-01-25]**

**Description**

**Domaine technique**

La présente invention se rapporte à un procédé d'analyse d'une interaction entre une première molécule et au moins une deuxième molécule liée à une particule au moyen de l'application d'une force sur cette particule, ainsi qu'à un dispositif d'analyse de cette interaction et à l'utilisation du procédé et du dispositif dans un criblage d'une molécule candidate pour le développement d'un médicament.

La présente invention trouve des applications notamment dans le domaine de la recherche comme outil de laboratoire.

Dans la description ci-dessous, les références entre crochets ([ ]) renvoient à la liste des références présentée à la fin du texte.

**Etat de la technique**

La mesure à haut débit des interactions biomoléculaires, en particulier pour les protéines mais aussi pour des molécules de poids moléculaire plus faible comme les oligonucléotides, est un enjeu important dans certains domaines d'application comme la recherche, le diagnostic ou le criblage pour la recherche de nouveaux médicaments.

Pour mesurer ces interactions, on peut mesurer par exemple la capacité et la cinétique d'association entre les biomolécules.

Parmi les techniques actuellement disponibles, la résonance plasmonique de surface est une technique largement employée qui permet de mesurer, sans marquage, si une protéine présente en solution interagit avec des ligands immobilisés sur une surface (Piehler et al., « New methodologies for measuring protein interactions in vivo and in vitro », Curr Opin Struct Biol. 2005 Feb;15(1):4-14, [1]). Cette technique est en outre réalisable avec un ligand fixé dans une bicouche lipidique artificielle (Cooper MA, « Advances in membrane receptor screening and analysis », J Mol Recognit. 2004 Jul-Aug;17(4):286-315, [2]). La détection est fondée sur le fait que la liaison de la protéine à la surface induit une modification de l'indice de réfraction. Ceci entraîne un déplacement de la fréquence de résonance du plasmon de surface, qui se traduit soit par un changement dans l'intensité réfléchie à un angle fixé, soit par un changement de l'angle auquel le maximum d'intensité réfléchie est observé. Un des inconvénients de cette technique est que la modification du signal lors de la liaison de petites molécules est très faible, et difficilement détectable.

Des solutions pour étudier les interactions entre molécules ont été recherchées dans le développement d'outils permettant, notamment en temps réel, soit d'exercer une force sur l'une des deux molécules soit de mesurer la force exercée ou produite par de petites structures moléculaires.

La pince optique est ainsi un outil permettant de nanomanipuler un objet et, en particulier, de lui appliquer une force sans le toucher, c'est-à-dire sans qu'il y ait de contact matériel avec l'objet (S. Chu, Laser Manipulation of Atoms and Particles, Science 253, 861 (1991) ([3])). Les pinces optiques utilisent le minimum d'énergie produit par un laser focalisé pour pouvoir déplacer une bille micrométrique et ainsi appliquer une force sur les objets accrochés à la bille. Ainsi une bille fixée à un lipide ou à une protéine membranaire a été piégée par une pince optique afin d'étudier son interaction avec la membrane suite à l'application d'une force via la pince optique (A. Pralle, P. Keller, E.-L. Florin, K. Simons, and J.K.H. Hörber, Sphingolipid-Cholesterol Rafts Diffuse as Small Entities in the Plasma Membrane of Mammalian Cells, J. Cell Biol. 148, 997-1007 (2000), ([4]), Kenichi Suzuki, Ronald E. Sterba, and Michael P. Sheetz, Outer Membrane Monolayer Domains from Two-Dimensional Surface Scanning Resistance Measurements, Biophys. J. 79 448-459 (2000) ([5])).

Une autre façon de manipuler une biomolécule est d'utiliser une pince magnétique. Par exemple, en accrochant spécifiquement une molécule d'ADN par ses extrémités entre une surface de verre et une microbille paramagnétique, il est possible de manipuler mécaniquement cette molécule en agissant sur la bille au moyen d'un champ magnétique produit par des aimants.

D'autres outils pour appliquer une force afin d'étudier l'interaction entre molécules ont été proposés : la microscopie à force atomique (AFM) (Moy, V. T., Florin, E.-L. & Gaub, H. E. Intermolecular forces and energies between ligands and receptors. Science 264, 257$\pm$259 (1994) ([6]), Hinterdorfer, P., Baumgartner, W., Gruber, H. J., Schilcher, K. & Schindler, H. Detection and localization of individual antibody-antigen recognition events by atomic force microscopy. Proc. Natl Acad. Sci. USA 93, 3477-3481 (1996) ([7])) et une technique appelée « biomembrane force probe » basée sur le déplacement mécanique d'une micropipette avec une capsule membranaire au bout dont la tension de membrane peut être modulée par la pression de succion de la micropipette (Evans, E., Ritchie, K. & Merkel, R. Sensitive force technique to probe molecular adhesion and structural linkages at biological interfaces. Biophys. J. 68, 2580-2587 (1995) ([8]), R. Merkel, P. Nassoy, A. Leung, K. Ritchie, E. Evans, Nature 397, 50-53 (1999) ([9])).

L'application d'une force au moyen de pinces optiques ou magnétiques ou au moyen d'une pointe AFM ou d'une micropipette implique des procédés complexes, qui nécessitent du matériel onéreux, notamment des lasers à forte puissance ou des aimants dans le cas des pinces, de l'électronique complexe dans le cas de l'AFM et des alignements difficiles dans le cas de la micropipette. En outre le matériel utilisé est volumineux et malaisé à déplacer, ce qui rend son usage difficile voire impossible dans un kit mobile. Par ailleurs, dans le cas de pinces optiques, l'énergie des lasers peut être absorbée par le milieu et dissipée en chaleur et provoquer une dénaturation de la molécule à étudier. De plus, ces techniques ne se prêtent pas ou pas facilement au multiplexage.

Des procédés ont été développés pour évaluer les interactions entre biomolécules sans utiliser ces dispositifs, notamment le laser.

Par exemple, le document WO 2009098272 ([10]) décrit l'évaluation des propriétés d'adhésion, de mouvement et de morphologie de cellules soumises à un flux. Le dispositif décrit est un dispositif de macroanalyse de dépôt de plaquettes ou de films protéiques sur une surface. Le dispositif décrit dans ce document ne permet cependant pas d'analyser des interactions au niveau moléculaire.

Le document WO2006009398 ([11]) décrit une méthode de détection d'interactions moléculaires, utilisable in vivo et in vitro, ainsi qu'un appareil microfluidique pour déterminer les interactions entre deux biomolécules, dans lesquels une des biomolécules est marquée, et dans lesquels les biomolécules sont mises en contact au moyen d'un flux appliqué à celles-ci. L'inconvénient de cette technique réside dans l'utilisation d'un champ magnétique pour exercer la force, ce qui demande une installation coûteuse. Par ailleurs, elle ne permet pas d'accès à la dynamique de l'interaction entre biomolécules puisque l'application de la force et la détection sont deux étapes distinctes.

WO 03/095973 décrit un réactif et son utilisation, comprenant une particule ayant une étiquette Raman et une partie de liaison spécifique liée à la particule. En présence d'un analyte cible, ce réactif peut former un complexe en liant l'analyte cible sur sa partie de liaison spécifique. Le complexe est généralement immobilisé sur un support solide. Il est traité avec un agent de coloration pour activer l'effet SERS après irradiation au laser. L'analyte cible peut être une molécule d'ADN, ou une protéine, un peptide, un médicament, de petites molécules, des virus, des anticorps, des carbohydrates et d'autres bio-molécules. La particule peut être une particule d'or, d'argent, de cuivre, de platine, un alliage or/argent, platine/or, cuivre/or ou une nanoparticule, éventuellement une nanoparticule métallique. Dans un mode de réalisation particulier, une molécule d'oligonucléotides liée a un support est mise en contact avec un échantillon comprenant des molécules d'oligonucléotides à tester, et avec un réactif tel que décrit ci-dessus, ce réactif comprenant une molécule d'oligonucléotides; dans des conditions rendant possible leur hybridation, afin de détecter la présence d'un polymorphisme dans l'échantillon d'oligonucléotides. Après la mise en contact des oligonucléotides, un lavage stringent est appliqué afin de détacher toute liaison non spécifique entre les oligo-nucléotides.

WO 2007/092909 décrit un capteur d'interactions entre molécules, comprenant au moins un complexe d'interaction moléculaire impliquant un liant dislocable qui possède une première extrémité et une seconde extrémité, la première extrémité étant couplée à une étiquette détectable et la seconde extrémité étant couplée à la surface d'un support; un détecteur conçu pour détecter l'étiquette et au moins un dispositif de lecture. L'étiquette détectable peut être une nanoparticule superparamagnétique et le détecteur est un dispositif magnétorésistant impliquant la mesure de la résistance à un champ magnétique. Le liant est disloqué par une molécule cible prédéterminée spécifique. Dans un mode de réalisation particulier, le détecteur comprend en outre un support microfluidique qui délivre un échantillon test au détecteur et génère un flux pour enlever une partie de la composition magnétique.

MIALON G. ET AL: "Luminescent oxide nanoparticles with enhanced optical properties", JOURNAL OF LUMINESCENCE, AMSTERDAM, NL, vol. 129, no. 12, décembre 2009, pages 1706-1710, décrit des nanoparticules luminescentes à base d'oxyde, ayant des propriétés optiques améliorées pour une utilisation comme marqueur de biomolécules. MASSON J -B. ET AL: "Inferring maps of forces inside cell membrane microdomains", PHYSICAL REVIEW LETTERS AMERICAN PHYSICAL SOCIETY USA, vol. 102, no. 4, janvier 2009, décrit la cartographie des forces exercées sur des biomolécules dans des membranes cellulaires. L'utilisation de nanoparticules inorganiques permet, via une fluorescence, de détecter des mouvements spontanés de biomolécules auxquelles elles sont fixées.

BEAUREPAIRE E. ET AL: "Functionalized luminescent oxide nanoparticles for sodium channel imaging at the single molecule level", PROCEEDINGS OF THE SPIE - THE INTERNATIONAL SOCIETY FOR OPTICAL ENGINEERING SPIE - USA, vol. 5705, no. 1, avril 2005, pages 192-198, décrit des nanoparticules à base d'oxyde, fonctionnalisées et luminescentes, pour la visualisation d'une seule molécule d'un canal sodique.

Le document Schmidt Brian J. et al. "Catch Strip Assay for the Relative Assessment of Two-Dimensional Protein Association Kinetics", Anal. Chem. 2008, 80 :944-950 [49] a proposé d'analyser l'interaction entre une première et une deuxième molécule grâce au déplacement observé d'une microparticule liées à la deuxième molécule et l'application d'un flux. Dans le cas où la particule est une microparticule, un nombre important de deuxièmes molécules sera lié à la microparticule. Dans cette situation, on ne peut observer que la moyenne entre un grand nombre d'interactions entre des premières et des deuxièmes molécules. Cette approche ne permet pas l'étude d'une paire unique de première et de deuxième molécule. S'il existe, par exemple, deux formes de premières molécules, M1 et M1*, ou deux formes de deuxièmes molécules M2 et M2*, cette approche ne permettra pas de distinguer des différences d'interaction entre M1-M2 et M1*-M2 ou entre M1-M2 et M1-M2*. De même, cette approche ne permet pas d'étudier l'interaction d'une seule première molécule avec son environnement suite à l'application d'une force via la deuxième molécule. En effet, l'approche de la référence Schmidt Brian J. et al., Anal. Chem. 2008, 80 :944-950 [49], permet d'étudier l'interaction d'un ensemble de molécules M1 « de nombre inconnu » avec leur environnement. Il est ainsi impossible de remonter à l'énergie d'interaction d'une seule molécule avec son environnement ou à la distribution de valeurs de l'énergie d'interaction entre deux molécules.

Disposer de ces informations est pourtant essentiel pour pouvoir comprendre la nature de ces interactions mais aussi pour un criblage performant d'une molécule candidate pour le développement d'un médicament.

Il existe donc un réel besoin d'un outil et d'un procédé permettant d'analyser une interaction entre molécules palliant ces défauts, inconvénients et obstacles de l'art antérieur, notamment un outil dont la mise en oeuvre est plus simple, plus pratique, moins onéreuse, compatible avec une étude simultanée de multiples interactions et suffisamment précise pour l'analyse, à l'échelle moléculaire, des interactions entre molécules.

En particulier, il existe donc un besoin d'un outil et d'un procédé permettant d'analyser une interaction entre molécules au niveau de la paire unique entre molécules.

## Description de l'invention

L'invention permet précisément de pallier les inconvénients de l'art antérieur et de répondre à ce besoin.

A l'issue d'importantes recherches, la Demanderesse a mis au point un procédé d'analyse d'une interaction entre molécules, dont la mise en oeuvre est simple, peu onéreuse, pratique, suffisamment précise pour analyser, à l'échelle moléculaire, des interactions entre molécules. Notamment, le procédé d'analyse ne requiert pas l'utilisation d'un laser ou d'un aimant ou d'une pointe AFM pour générer une force, ni d'instrument complexe pour la lecture des résultats. Elle permet en outre d'analyser des interactions entre molécules sans les détériorer ni les dénaturer. L'invention permet en outre d'analyser l'interaction d'une très petite molécule avec un ligand, ou de très petites molécules entre elles. De plus, l'invention permet d'étudier un nombre d'interactions réduit, voir une seule interaction, entre une première molécule et une deuxième molécule. Elle présente également une capacité de multiplexage et permet, en particulier, d'étudier simultanément et individuellement un grand nombre d'interactions entre paires uniques de première et deuxième molécules.

Ainsi, un premier objet de l'invention se rapporte à un procédé d'analyse d'une interaction entre au moins une première molécule et au moins une deuxième molécule liée à une particule, comprenant les étapes suivantes :

- mettre en contact la première molécule et la deuxième molécule liée à la particule dans des conditions rendant possible leur interaction,
- appliquer un flux liquide déterminé sur la particule liée à la deuxième molécule,
- observer un déplacement de la particule liée à la deuxième molécule sous l'action du flux appliqué,
- analyser l'interaction en fonction du déplacement observé et du flux appliqué,

la particule ayant une résistance hydrodynamique supérieure à celle de la première et/ou de la deuxième molécule, un nombre de Péclet massique supérieur à 1 et une dimension de taille nanométrique comprise entre 1 et 100 nm.

Un autre objet de l'invention se rapporte à un dispositif d'analyse d'une interaction entre une première molécule et au moins une deuxième molécule, comprenant : un moyen d'application d'un flux liquide déterminé, un support d'accueil d'une première molécule, une particule capable de lier une deuxième molécule susceptible d'interagir avec la première molécule ladite particule ayant une dimension de taille nanométrique comprise entre 1 et 100 nm, un moyen d'observation du déplacement de la particule, et un moyen de contrôle du flux liquide déterminé, dans lequel la particule a une résistance hydrodynamique supérieure à celle de la première et/ou de la deuxième molécule, et un nombre de Péclet massique supérieur à 1.

Dans la présente description, « une molécule » signifie « au moins une molécule », c'est-à-dire une ou plusieurs molécules. Lorsqu'il s'agit de plusieurs molécules, elles peuvent être identiques ou différentes. La première et la deuxième molécule peuvent être identiques ou différentes. Il peut s'agir également d'une première molécule M1 présente sous deux ou plusieurs formes différentes, par exemple, M1 et M1*, ou d'une deuxième molécule M2 présente sous deux ou plusieurs formes différentes, par exemple, M2 et M2*. Dans ce cas précis, il s'agira de détecter et comparer pendant la même expérience l'affinité d'une unique première molécule M1 pour une unique deuxième molécule M2 par rapport à une deuxième molécule M2* ou détecter et comparer pendant la même expérience l'affinité d'une unique deuxième molécule M2 pour une unique première molécule M1 par rapport à une première molécule M1*.

Il peut s'agir d'une molécule organique ou inorganique. Lorsqu'il s'agit d'une molécule organique, il peut s'agir d'une molécule synthétique ou d'une molécule d'origine biologique. Dans le cas de molécules présentes sous deux ou plusieurs formes différentes, M et M*, il peut s'agir, par exemple, de deux ou plusieurs conformations différentes de la même protéine ou deux ou plusieurs différents types d'hélices ADN.

En d'autres termes, la présente invention peut avantageusement être utilisée pour l'analyse de tout type d'interaction moléculaire, et donc pour tout type de molécules interagissant. Il peut s'agir d'une interaction de type organique/organique, organique/inorganique ou inorganique/inorganique.

On entend par « interaction moléculaire », au sens de la présente invention, toute liaison non covalente et réversible. L'interaction peut être spécifique ou non spécifique. Dans le cas d'une interaction spécifique, il peut s'agir par exemple d'une interaction antigène-anticorps, ligand-récepteur, ou enzyme-substrat, cette liste n'étant pas limitative. Dans le cas d'une interaction non-spécifique, il peut s'agir par exemple d'une interaction entre une molécule biologique et une surface.

Lorsque la première molécule et/ou la deuxième molécule est(sont) une molécule organique, elle(s) peut(peuvent) être choisie(s) par exemple dans le groupe comprenant un acide aminé, un peptide, une protéine, un nucléoside, un nucléotide, un acide nucléique, un acide gras, un lipide, simple ou complexe, un sucre, un monosaccharide, un polysaccharide, un phospholipide, un glycolipide, une glycoprotéine, une lipoprotéine, une glycolipoprotéine, ou tout autre molécule d'origine biologique, interagissant ou susceptible d'interagir avec une autre molécule. Dans le cas où la molécule est une molécule d'acide nucléique, il peut s'agir d'ADN (acide désoxyribonucléique) ou d'ARN (acide ribonucléique). Dans le cas où la molécule est une molécule peptidique ou protéique, il peut s'agir d'une molécule choisie dans le groupe comprenant un médicament candidat, une molécule d'une membrane cellulaire, une toxine, un anticorps, un composant matriciel de la peau, comme par exemple le collagène ou la fibronectine, un récepteur, notamment un récepteur membranaire. Il peut s'agir également de systèmes multimoléculaires, par exemple un ribosome, un protéasome, un apoptosome, un centrosome, ou des compartiments cellulaires tels qu'une mitochondrie, un endosome, un lysosome, un phagosome, une vésicule, voire également une cellule, un organisme unicellulaire, comme par exemple une bactérie, une levure, un champignon, une algue unicellulaire ou un protozoaire. Il peut également s'agir d'un système multicellulaire, comme par exemple une culture cellulaire, un épithélium, un fragment de tissu ou une colonie bactérienne.

Lorsque la molécule est une molécule inorganique, il peut s'agir de toute molécule inorganique connue de l'homme du métier ou d'une surface inorganique, comme par exemple une surface de polymère, une surface métallique, une surface fluorée, une surface de silicium. A titre d'exemple, la première molécule peut être un récepteur d'une membrane cellulaire, et la deuxième molécule peut être un ligand du récepteur ou une molécule susceptible de se lier à ce récepteur.

La première ou deuxième molécule peut avoir avantageusement un poids moléculaire allant de 300 à 300 000 g.mol$^{-1}$. Avantageusement, il peut s'agir d'une petite molécule, dont le poids moléculaire va de 100 à 4000 g.mol$^{-1}$.

La première molécule peut être liée à un support d'accueil ou être une molécule du support d'accueil. Le support d'accueil peut être toute surface permettant d'accueillir la première molécule. De préférence, cette surface permet la fixation de la première molécule sur celle-ci, de préférence en conservant la conformation de la molécule. En effet, par exemple lorsqu'il s'agit d'une protéine qui doit interagir avec la deuxième molécule, il est important que les zones d'interaction de la protéine avec la deuxième molécule ne soient pas déformées par la fixation de la protéine sur la surface. Avantageusement, la première molécule est liée au support de manière suffisamment forte pour ne pas être détachée du support par le flux liquide déterminé lors de la mise en oeuvre du procédé de l'invention.

La fixation de la première molécule peut être directe ou indirecte. Elle est directe lorsque la première molécule interagit directement avec la surface, soit parce que la surface a été fonctionnalisée au préalable, soit parce qu'elle présente des propriétés chimiques intrinsèques qui permettent de fixer la première molécule. Elle est indirecte lorsqu'un bras espaceur ou une structure multi-moléculaire, par exemple un fragment de paroi cellulaire, est fixé sur la surface.

Avantageusement, le support peut comprendre une ou plusieurs zones de liaison sur laquelle ou lesquelles est (sont) liée(s) la première molécule. Dans le cas où le support comporte une seule zone de liaison, un ou plusieurs types de première molécule peut être liée à la zone. Dans le cas où le support comporte plusieurs zones de liaison, chaque zone peut comprendre un ou plusieurs types de première molécule. Avantageusement, la première molécule peut être différente d'une zone à l'autre. La réalisation de plusieurs zones de liaison peut se faire à l'aide d'un microdispenseur (également appelé spotter) (I. Barbulovic-Nad, M. Lucente, Y. Sun, M. Zhang, A. R. Wheeler, M. Bussmann, Bio-Microarray Fabrication Techniques-A Review, Critical Reviews in Biotechnology, 26:237-259, 2006 ([12])) qui permet le dépôt de différentes molécules en des emplacements contrôlés d'une surface.

Avantageusement, le support peut permettre de réaliser plusieurs procédés d'analyse simultanément ou de manière séquentielle, par exemple avec différents types de particules et/ou différents types de première molécule et/ou différents types de deuxième molécule et/ou de premières molécules légèrement différentes l'une de l'autre et/ou de deuxièmes molécules légèrement différentes l'une de l'autre.

Le support peut être un support du type de ceux utilisés dans les puces à ADN, ou dans les puces à protéines, cette liste n'étant pas limitative. Il peut s'agir par exemple de supports disponibles dans le commerce, comme les supports commercialisés par Affymetrix (http://www.affymetrix.com/estore/).

Toute matière connue de l'homme du métier permettant d'obtenir ces caractéristiques peut être utilisée, comme par exemple le verre, le silicium, le plastique, comme du polystyrène, du polydiméthylsiloxane ou du polyméthacrylate, cette liste n'étant pas limitative. Le support peut être fabriqué selon toute méthode connue de l'homme du métier. Il peut s'agir par exemple de supports en verre, par exemple ceux commercialisés par VWR : http://fr.vwr.com/app/Home.

Il peut en outre s'agir d'une surface transparente ou opaque.

On entend par « particule » au sens de la présente invention, tout assemblage d'atomes formant un objet organique ou inorganique. La particule permet d'appliquer une force sur la deuxième molécule. Avantageusement, la particule peut être observée ou visualisée, ce qui permet d'observer ou de visualiser simultanément la position ou le déplacement de la deuxième molécule.

On entend par « résistance hydrodynamique », au sens de la présente invention, la force résultant de l'ensemble des contraintes exercées par l'écoulement du flux liquide déterminé sur la particule. Les dimensions de la particule sont telles qu'elle a une résistance hydrodynamique supérieure à celle de la première et/ou de la deuxième molécule. Avantageusement, la résistance hydrodynamique de la particule peut permettre le déplacement, sous l'effet d'un flux liquide contrôlé, de la particule et de la deuxième molécule.

On entend par « nombre de Péclet massique », au sens de la présente invention, le rapport entre le temps caractéristique de diffusion d'une masse et le temps de convection de cette masse. Il est égal à $vL/D$, où $v$ est la vitesse caractéristique de l'écoulement, $L$ la taille caractéristique de la particule et $D$ le coefficient de diffusion de la particule. Le nombre de Péclet massique de la particule est bien supérieur à 1. Il peut par exemple être compris entre 1 et 10000. Ces caractéristiques du nombre de Péclet massique impliquent que la particule possède une taille suffisante pour que son déplacement dû au mouvement Brownien soit négligeable par rapport à l'effet d'entraînement par le flux déterminé.

La particule peut être une microparticule, c'est-à-dire avoir au moins une dimension de taille micrométrique. Par exemple la micro particule a une taille comprise entre 1 et 100 $\mu$m, par exemple entre 10 et 80 $\mu$m, ou encore entre 20 et 70 $\mu$m, ou entre 30 et 50 $\mu$m.

La particule a une dimension de taille nanométrique comprise entre 1 et 100 nm.

La particule peut être toute particule connue de l'homme du métier, comme par exemple de particules de verre, de latex, de polystyrène, de métaux, par exemple le carbone, or, argent, cuivre, et de matériaux magnétiques, cette liste n'étant pas limitative.

Il peut s'agir également de nanoparticule d'origine naturelle ou synthétique, comme par exemple un métal, un semiconducteur, un oxyde métallique, un polymère, une nanosphère, cette liste n'étant pas limitative.

Il est généralement admis dans la communauté des utilisateurs de nanoparticules que le milieu aqueux environnant une particule attachée à une molécule de la membrane cellulaire n'a pas d'effet sur son mouvement. En l'absence de mouvement contrôlé du fluide, il a été montré que le mouvement de la nanoparticule n'est pas contrôlé par le fluide environnant mais par le comportement de l'objet auquel elle est attachée. En effet, comme la viscosité de la membrane est bien plus importante que la viscosité de l'eau et malgré la taille plus importante de la nanoparticule par rapport à celle d'une molécule membranaire (lipide ou protéine membranaire), le mouvement de l'ensemble est déterminé par le mouvement de la molécule membranaire.

Par ailleurs, la prédiction de la force hydrodynamique exercée sur une nanoparticule n'est pas évidente et un calcul rapide d'ordre de grandeur basé sur la loi de Poiseuille et de Stokes, compte tenu du fait que la vitesse est nulle à la paroi et que la nanoparticule et son système d'attachement sont en général très petits devant toutes les autres échelles des écoulements, indique des forces trop faibles pour avoir un effet significatif sur l'interaction entre la première et la deuxième molécule ou sur le comportement de la première molécule. Contrairement à ce que ces faits laissent penser, il est possible d'exercer une force, à l'aide d'une nanoparticule dans un flux, suffisamment importante pour, par exemple, déplacer une molécule membranaire à l'intérieur de la membrane cellulaire. En effet, près de la surface du canal, la loi de Stokes n'est plus valide et les forces hydrodynamiques exercées sont plus importantes que celles prédites par la loi de Stokes : voir A. J. Goldman, R. G. Cox, H. Brenner, Slow viscous motion of a sphere parallel to a plane wall-I Motion through a quiescent fluid, Chem. Eng. Sci. 22 637-651 (1967) ([16]), A. J. Goldman, R. G. Cox, H. Brenner, Slow viscous motion of a sphere parallel to a plane wall-II Couette flow Chem. Eng. Sci. 22 653-660 (1967) ([17]), E. Lauga, T. M. Squires, Brownian motion near a partial-slip boundary: A local probe of the no-slip condition, Phys. Fluids 17, 103102 (2005) ([18]), J. Happel and H. Brenner, Low Reynolds Number Hydrodynamics, p286-340 (Martinus Nijhoff, The Hague, 1983) ([19]).

La particule peut avoir au moins une de ses dimensions de taille supérieure à celle de la deuxième molécule. La particule peut avoir au moins une dimension comprise entre cinq et cent fois celle de la deuxième, par exemple entre dix et vingt fois.

La particule peut être d'une forme adaptée à être entraînée par le flux liquide déterminé. La particule peut être de forme substantiellement sphérique, par exemple substantiellement ronde ou ovale. Il peut dans ce cas s'agir d'une bille. Alternativement, la particule peut être de forme non sphérique, par exemple ovoïde, cylindrique ou discoïde.

La particule peut être toute molécule adaptée disponible dans le commerce, par exemple chez Invitrogen pour les Quantum Dots à base de semiconducteur (http://www.invitrogen.com/site/us/en/home/brands/Molecu lar-Probes/Key-Molecular-Probes-Products/Qdot.html), ou chez British Biocell International pour les nanoparticules d'or (http://www.britishbiocell.co.uk/products/goldreagents.asp?navid=2), ou être fabriquée par tout moyen connu de l'homme du métier, par exemple, pour les nanoparticules dopées par des terres rares, selon la méthode décrite par Huignard et al. (Huignard, A.; Gacoin, T.; Boilot, J.-P. Chem. Mat. 2000, 12, 1090 ([13])).

De préférence, la liaison entre la particule et la deuxième molécule est suffisamment stable pour ne pas être rompue par le flux liquide déterminé. La particule et la deuxième molécule peuvent être liées par tout moyen connu de l'homme du métier, par exemple par une liaison chimique faible, comme par exemple par force électrostatique, force de Van der Waals, liaison hydrogène, liaisons hydrophobes, ou encore par une liaison chimique forte, comme par exemple par liaison ionique, covalente ou métallique, ou encore au moyen d'un agent de couplage, comme par exemple des agents de couplage portant des fonctions doubles permettant de se fixer d'une part sur des fonctions (par exemple fonctions amines ou fonctions acides carboxyliques) présentes à la surface de la particule et, d'autre part, sur des fonctions de la deuxième molécule (par exemple fonctions amines ou fonctions sulfhydryles). De tels agents de couplage sont disponibles dans le commerce, comme par exemple chez Pierce (http://www.piercenet.com/Products/Browse.cfm?fldID=0203). La particule et la deuxième molécule peuvent également être liées en utilisant par exemple des interactions biologiques à forte affinité, comme l'interaction biotine-streptavidine (ou l'interaction ligand-récepteur ou l'interaction anticorps-antigène), et un couplage à plusieurs étapes, c'est-à-dire d'abord couplage de la streptavidine (ou de la biotine) à la particule et couplage de la biotine (ou de la streptavidine) à la deuxième molécule et ensuite mise en interaction des deux produits de couplage.

La première et la deuxième molécule peuvent être mises en contact dans le dispositif d'analyse de l'invention. Le dispositif de l'invention peut, à cet égard, comprendre tout moyen permettant la mise en contact de la première et de la deuxième molécule. Il peut s'agir d'un canal d'une géométrie adaptée à la mise en contact de la première et de la deuxième molécule. La géométrie du canal peut par exemple être droite, en forme de T ou en forme de Y, ou bien être d'une géométrie plus complexe afin de pouvoir ajouter des réactants de manière indépendante. La géométrie pourra être aisément déterminée par l'homme du métier en fonction du nombre de réactants introduits, ou du nombre de première et/ou deuxième molécules différentes introduites.

Avantageusement, le dispositif peut posséder au moins une entrée, de manière à pouvoir y introduire la première molécule, la deuxième molécule liée à la particule, et le flux liquide déterminé. Le dispositif peut en outre posséder autant d'entrées qu'en nécessite le nombre de première et/ou deuxième molécules différentes injectées dans le dispositif, ou autant d'entrées qu'en nécessite le nombre d'analyses effectuées simultanément ou de manière séquentielle. Par exemple, le dispositif peut posséder deux entrées : l'une pour l'injection des particules couplées avec la deuxième molécule, et l'autre pour appliquer le flux liquide. La présence de deux entrées distinctes peut permettre d'obtenir un flux qui ne contient pas de particules liées à la deuxième molécule. La présence de deux entrées distinctes peut ainsi permettre d'augmenter le ratio signal/bruit de fond par rapport à un dispositif avec une seule entrée.

Avantageusement, le dispositif peut comprendre en outre une sortie. La sortie peut être connectée à un réservoir. Le réservoir peut permettre de modifier la direction du flux sans risque d'introduire des bulles dans le dispositif. Le réservoir n'est avantageusement requis que dans les modes de réalisation impliquant un changement de direction du flux.

Les dimensions du canal peuvent être choisies en fonction de la nature et de la taille de la première et/ou de la deuxième molécule et/ou de la particule. Par exemple, le canal peut avoir une hauteur, c'est-à-dire une distance entre le support d'accueil de la première molécule et la paroi supérieure du canal, suffisamment élevée pour permettre l'étude de l'interaction de la première et de la deuxième molécule. La hauteur peut donc dépendre des dimensions de la première et/ou de la deuxième molécule. Par exemple, dans le cas où la première molécule est une cellule, un canal de hauteur insuffisante peut avoir pour conséquence des cellules trop confinées et un impact négatif sur leur physiologie. La hauteur du canal est limitée par la gamme de vitesse voulue du fluide au niveau de la particule en fonction des moyens de contrôle de l'écoulement utilisés : plus le canal est haut, plus la vitesse est faible. A titre d'exemple, le canal peut avoir une hauteur comprise entre 20 et 70 $\mu$m, par exemple d'environ 30 $\mu$m, ou encore environ 40 $\mu$m, environ 50 $\mu$m, ou environ 60 $\mu$m. Par exemple, si la première molécule est une cellule, la hauteur du canal pourra être d'environ 50 $\mu$m. La largeur du canal doit être suffisamment grande pour permettre d'observer la quantité voulue de la première molécule. Avantageusement, le ratio hauteur/largeur doit être suffisamment petit pour permettre la création d'un flux liquide déterminé, correspondant à un flux laminaire. Le ratio peut être par exemple compris entre 1/100 et 1/2. Le canal peut avoir une largeur comprise entre 100 $\mu$m et 2 mm, par exemple environ 500 $\mu$m. La longueur du canal peut être suffisante pour éviter les perturbations du flux liquide déterminé lorsque celui-ci est soumis à une entrée et/ou à une sortie de liquide. Avantageusement, la longueur peut être suffisante pour assurer des profils hydrodynamiques de lignes de courant parallèles aux parois du canal sur une zone suffisamment longue du canal. Le canal peut avoir une longueur comprise entre 1 mm et 10 cm, par exemple d'environ 1,5 cm. La longueur peut permettre de lier un nombre de premières molécules suffisamment grand pour permettre l'analyse de l'interaction. La longueur du canal peut être limitée dans les cas où il repose sur un appareil optique, par exemple sur une lamelle optique.

Les conditions de la mise en contact de la première et de la deuxième particule sont facilement déterminables par l'homme du métier en fonction de la première et de la deuxième molécule.

On entend par « flux liquide », au sens de la présente invention, tout écoulement liquide. Avantageusement, le flux permet d'appliquer une force sur la particule. Il peut en outre permettre d'entraîner la particule. Le flux peut être unidirectionnel ou avoir plusieurs directions, par exemple alternées. Avantageusement, le flux peut être laminaire. Il peut s'agir d'un écoulement liquide d'une solution, par exemple une solution aqueuse, une solution aqueuse saline de pH fixé par un tampon phosphate (PBS) ou par un tampon HEPES, un milieu de culture cellulaire ou toute autre solution nécessaire à l'étude de la première et/ou la deuxième molécule dans de bonnes conditions, d'une dispersion. Avantageusement, le flux liquide déterminé peut contenir des objets, flottants dans le flux et déplacés par le flux. Avantageusement, la mesure de la vitesse de ces objets peut permettre de mesurer la vitesse du fluide autour de la particule. Cette mesure peut se faire dans une étape séparée de celle de l'analyse de l'interaction entre première et deuxième molécule.

On entend par « flux déterminé », au sens de la présente invention, tout flux dont les caractéristiques sont telles qu'elles permettent un contrôle de la force appliquée sur la particule. En d'autres termes, le flux déterminé peut être un flux dont les caractéristiques permettent un choix de l'amplitude et de la direction de la force appliquée sur la particule. Avantageusement, le flux déterminé est tel qu'il peut permettre d'appliquer une force sur la particule provoquant le déplacement de celle-ci. La force est telle qu'elle ne provoque pas un détachement de la première molécule du support ni une détérioration du support. Les caractéristiques du flux peuvent être déterminées au moyen du contrôle d'au moins un des paramètres choisi dans le groupe comprenant le débit ou la vitesse du liquide, la viscosité du liquide, et de la pression appliquée sur le liquide.

La viscosité du liquide peut être déterminée par toute méthode connue de l'homme du métier, comme par exemple à l'aide d'un viscosimètre capillaire en mesurant le débit engendré par une différence de pression aux deux extrémités d'un tube capillaire (loi de Poiseuille) ou à l'aide d'un viscosimètre Couette-Plan (P. Cousson C. Ancey, Rhéophysique des pâtes et des suspensions, Ed. EDP Sciences (1999) ([14]).

La vitesse du flux liquide déterminé peut être continue, c'est-à-dire de valeur constante. Alternativement, la vitesse peut être discontinue, c'est-à-dire avoir plusieurs valeurs. Dans ce cas, la vitesse peut croître ou décroître de manière linéaire. Alternativement, la vitesse peut croître ou décroître par paliers, avec des périodes entre chaque palier qui pourront être aisément déterminées par l'homme du métier en fonction des informations qu'il souhaite recueillir.

La vitesse du flux liquide déterminé peut être mesurée par toute méthode connue de l'homme du métier. Avantageusement, la vitesse du flux liquide déterminé peut être déterminée précisément à chaque instant, au moyen d'une méthode connue de l'homme du métier, comme par exemple un calcul de la vitesse en fonction de la distance de la paroi selon la loi de Poiseuille. S'il s'agit d'un flux électro-osmotique, la vitesse est constante à l'intérieur du canal et dépend du champ électrique appliquée et de la géométrie du canal (P. Tabelling, Introduction à la microfluidique, p178-202 Editions Belin (2003) ([15]). La vitesse du flux liquide déterminé à la position de la particule peut être calculée à partir du débit et/ou de la vitesse du flux en amont et/ou en aval de la particule. Dans ce cas, la particule est supposée ne modifier que faiblement le flux et ne pas influer sur les autres particules et sur la mesure du débit. Cette méthode de calcul permet en outre de réaliser des comparaisons quantitatives entre plusieurs expériences. Alternativement, la vitesse peut être mesurée par visualisation de marqueurs, flottants, non liées et n'interagissant pas avec la première et/ou la deuxième molécule, véhiculées par le flux. Différents marqueurs flottants, par exemple de différentes tailles, peuvent être injectés dans le flux. Par exemple, des billes fluorescentes peuvent être utilisées à cet effet.

Le flux liquide déterminé peut être tel qu'il permet d'appliquer à la particule une force F égale à $6\pi\mu Rv$, dans laquelle $\mu$ représente la viscosité du fluide, R le rayon hydrodynamique de la particule et v la vitesse du fluide à la position de la particule, ou une force F égale à $6\pi\mu Rv$ et corrigée pour les effets de proximité d'une paroi. La vitesse du fluide peut être mesurée en amont ou en aval de la particule, à une distance grande devant la particule, mais petite devant les autres modifications du flux. Dans le cas d'une particule sphérique solide de quelques dizaines de nanomètres, son rayon est très proche de son rayon hydrodynamique. Dans le cas d'une particule de très petite taille ou d'une particule non sphérique, son rayon hydrodynamique peut être déterminé, par exemple par la diffusion dynamique de la lumière. Des appareils permettant de mesurer le rayon hydrodynamique de particules ou de molécules par diffusion dynamique de la lumière sont commercialisés entre autres par la société Malvern (http://www.malvern.com/zetasizer). Dans le cas où la particule est située à une distance de la paroi du canal comparable à son rayon hydrodynamique, des facteurs correctifs notamment liés à la distance de la particule à la paroi, la géométrie de la particule, et les caractéristiques du flux (viscosité, vitesse), peuvent être appliquées à la formule ci-dessus, par exemple suivant les techniques citées dans les documents suivants : A. J. Goldman, R. G. Cox, H. Brenner, Slow viscous motion of a sphere parallel to a plane wall-I Motion through a quiescent fluid, Chem. Eng. Sci. 22 637-651 (1967) ([16]), A. J. Goldman, R. G. Cox, H. Brenner, Slow viscous motion of a sphere parallel to a plane wall-II Couette flow Chem. Eng. Sci. 22 653-660 (1967) ([17]), E. Lauga, T. M. Squires, Brownian motion near a partial-slip boundary: A local probe of the no-slip condition, Phys. Fluids 17, 103102 (2005) ([18]), J. Happel and H. Brenner, Low Reynolds Number Hydrodynamics, p286-340 (Martinus Nijhoff, The Hague, 1983) ([19]).

Le flux liquide déterminé peut être appliqué, et ses paramètres contrôlés, par tout moyen connu de l'homme du métier, par exemple tout dispositif utilisé en microfluidique pour contrôler un écoulement. L'écoulement peut, par exemple, être appliqué via une seringue montée sur un pousse-seringue, via un dispositif de contrôle de la pression ou via une pompe péristaltique.

On entend par « déplacement », au sens de la présente invention, tout changement de position spatiale de la particule par rapport à l'instant où la première et la deuxième molécule entrent en interaction. Avantageusement, la position de la particule est mesurée à l'instant où la première et la deuxième molécule entrent en interaction, et éventuellement au moment où le flux liquide déterminé commence à être appliqué. Avantageusement, la position de la particule peut être mesurée à intervalles réguliers avant et après l'application du flux liquide déterminé. Avantageusement, le déplacement de la particule peut s'accompagner du déplacement de la deuxième molécule. Avantageusement, le déplacement de la particule peut provoquer la rupture de l'interaction entre la première et la deuxième molécule.

Le déplacement ou la position de la particule peut être observé grâce aux propriétés physiques et/ou chimiques de la particule. Les propriétés physiques observables de la particule peuvent être son absorption, sa réflexion ou sa diffusion de la lumière, sa fluorescence, ses propriétés magnétiques ou électriques. Les propriétés chimiques de la particule peuvent par exemple donner lieu à une réaction avec un substrat menant à la génération d'un produit fluorescent ou absorbant. Il peut s'agir de chimiluminescence ou de bioluminescence.

Le déplacement de la particule liée à la deuxième molécule sous l'action du flux appliqué peut être observé par tout moyen connu de l'homme du métier. Il peut par exemple s'agir d'une technique choisie dans le groupe comprenant une méthode optique, une méthode chimique, une méthode électrochimique et une méthode magnétique (Jin-Woo Choi, Kwang W. Oh, Jennifer H. Thomas, William R. Heineman, H. Brian, Halsall, Joseph H. Nevin, Arthur J. Helmicki H. Thurman Henderson and Chong H. Ahn, An integrated microfluidic biochemical détection system for protein analysis with magnetic bead-based sampling capabilities, Lab Chip, 2002, 2, 27-30 ([20]), Maria A. Schwarz and Peter C. Hauser, Récent developments in détection methods for microfabricated analytical devices, Lab on a Chip, 2001, 1, 1-6 ([21]), Jun Yang. Jianlong Zhao. Mengsu Yang, Optical and electrochemical détection techniques for cell-based microfluidic systems, Anal Bioanal Chem (2006) 384: 1259-1268 ([22])).

La méthode optique peut être par exemple une méthode choisie dans le groupe comprenant une méthode de mesure d'un signal de fluorescence, une méthode d'analyse par résonance plasmonique de surface (Homola J, "Présent and future of surface plasmon résonance biosensors", ANALYTICAL AND BIOANALYTICAL CHEMISTRY 377, 528-53 (2003) ([23])), une méthode comprenant l'utilisation d'une pince optique (Molloy JE, Padgett MJ, "Lights, action: optical tweezers", CONTEMP. PHYS. 43, 241-258 (2002) ([24])), une méthode comprenant l'utilisation d'un laser ou de lumière blanche, cette liste n'étant pas limitative. Dans le cas où la méthode optique est une méthode d'analyse par résonance plasmonique de surface, le support peut être transparent.

Avantageusement, la méthode optique peut être une méthode choisie dans le groupe comprenant : une détection de l'absorption (D. Boyer, P. Tamarat, A. Maali, B. Lounis, M. Orrit, "Photothermal Imaging of Nanometer-Sized Metal Particles Among Scatterers", Science 297, 1160 (2002) ([25]), D. Lasne, G. A. Blab, S. Berciaud, M. Heine, L. Groc, D. Choquet, L. Cognet, B. Lounis, "Single Nanoparticle Photothermal Tracking (SNaPT) of 5-nm Gold Beads in Live Cells", Biophys. J. 91, 4598-4604 (2006) ([26]), Arbouet A, Christofilos D, Del Fatti N, Vallee F, Huntzinger JR, Arnaud L, Billaud P, Broyer M, "Direct measurement of the single-metal-cluster optical absorption", Phys. Rev. Lett. 93, 127401 (2004) ([27]), Muskens OL, Del Fatti N, Vallee F, Huntzinger JR, Billaud P, Broyer M, "Single metal nanoparticle absorption spectroscopy and optical characterization", Appl. Phys. Lett. 88, 063109 (2006) ([28])), de la réflexion, de la diffusion (De Brabander, M., R. Nuydens, G. Geuens, M. Moeremans, and J. de Mey, "The use of submicroscopic gold particles combined with video contrast enhancement as a simple molecular probe for the living cell". Cell Motil. Cytoskeleton. 6:105-113 (1986) ([29])), de la diffraction, du changement de l'indice de réfraction (D. Boyer, P. Tamarat, A. Maali, B. Lounis, M. Orrit, "Photothermal Imaging of Nanometer-Sized Metal Particles Among Scatterers", Science 297, 1160 (2002) ([30]), D. Lasne, G. A. Blab, S. Berciaud, M. Heine, L. Groc, D. Choquet, L. Cognet, B. Lounis, "Single Nanoparticle Photothermal Tracking (SNaPT) of 5-nm Gold Beads in Live Cells", Biophys. J. 91, 4598-4604 (2006) ([31]), Homola J, "Present and future of surface plasmon resonance biosensors" ([32])) et/ou l'utilisation d'un émetteur de fluorescence, d'un moyen d'excitation et d'un moyen de détection de la fluorescence (M. J. Saxton, K. Jacobson, "SINGLE-PARTICLE TRACKING:Applications to Membrane Dynamics", Annu. Rev. Biophys. Biomol. Struct.. 26, 373-99 (1997) ([33])).

En d'autres termes, l'observation peut être réalisée par une méthode optique choisie dans le groupe comprenant l'utilisation d'un émetteur de fluorescence et d'un moyen de détection de la fluorescence, une détection de l'absorption, une détection de la réflexion, une détection de la diffusion et une détection de la diffraction.

Dans le cas d'une détection du déplacement de la particule par une méthode de fluorescence, l'émetteur de fluorescence peut être la particule elle-même ou un marqueur associé à la particule et/ou à au moins une molécule choisie parmi la première molécule et la deuxième molécule. De préférence, l'émetteur de fluorescence peut être la particule elle-même ou un marqueur associé à la particule et/ou la deuxième molécule. La fluorescence peut se traduire par une ou plusieurs longueurs d'onde d'émission. Avantageusement, l'émetteur de fluorescence peut être tout type de molécule connue de l'homme du métier, possédant la propriété d'absorber de l'énergie lumineuse et de la restituer rapidement sous forme de fluorescence. Cet émetteur peut être un fluorophore organique ou une particule inorganique. Il peut s'agir par exemple d'un émetteur choisi parmi les Quantum Dots, les nanoparticules dopées par des ions lanthanides, les nanodiamants, les fluorophores organiques comme la rhodamine, le FITC (fluorescéine), les cyanines, l'Alexa, la dopamine ou la chromomycine A, les protéines fluorescentes (comme la GFP, YGF, DsRed), les particules inorganiques contenant des fluorophores organiques.

Dans le cas où l'émetteur de la fluorescence n'est pas la particule elle-même, le marquage de la particule ou de la deuxième molécule par l'émetteur de fluorescence peut être réalisé par toute technique connue de l'homme du métier. Il peut s'agir par exemple des techniques de marquage décrites dans les documents Chen et al. Nano Letters, vol. 7, No. 3, 690-696, 2007 ([33]), Bek et al., Nano Letters, vol. 8, No. 2, 485-490, 2008 ([34]), WO2007036544 ([35]) ou Aslan et al., Current Opinion in Chemical Biology, 9 :538-544, 2005 ([36]).

Avantageusement, la fluorescence peut permettre d'obtenir un ratio signal/bruit élevé.

La position de la particule peut être visualisée par imagerie, par exemple au moyen d'un microscope champ large, par exemple disponible dans le commerce chez Zeiss, Olympus, Nikon. Il peut être équipé d'une caméra CCD ou EM-CCD ou CMOS, par exemple celles disponibles dans le commerce chez Princeton Instruments, Andor, Hamamatsu. La position peut également être visualisée par une méthode possédant une résolution axiale, comme l'excitation à deux-photons ou l'excitation non linéaire (W. R. Zipfel, R. M. Williams, W. W.Webb, Nonlinear magic: multiphoton microscopy in the biosciences, Nat. Biotech. 21, 1369 (2003) ([37]) ou la réflexion interne totale (TIRF) (A. L. Stout and D. Axelrod, Evanescent field excitation of fluorescence by epi-illumination microscopy, Appl. Opt. 28, 5237 (1989) ([38]), N. L. Thompson and B. C. Lagerholm, Total internal reflection fluorescence: applications in cellular biophysics, Curr. Opin. Biotech., 8, 58-64 (1997) ([39])).

La position de la particule peut être déterminée au moyen de l'ajustement de l'image avec une Gaussienne à deux dimensions qui fournit le centre de la particule ou avec toute autre technique, telle que la corrélation entre images, utilisée dans le domaine du suivi de molécules/particules uniques (M. K. Cheezum, W. F. Walker, and W. H. Guilford, Quantitative Comparison of Algorithms for Tracking Single Fluorescent Particles, Biophys. J. 81 2378-2388 (2001) ([40])). Une précision supérieure à la résolution du microscope optique, limitée par la diffraction de la lumière, peut être obtenue et dépend du ratio signal/bruit et des caractéristiques du système de détection (fonction de réponse, taille des pixels de la caméra) (R. E. Thompson, D. R. Larson, and W. W. Webb, Précise Nanometer Localization Analysis for Individual Fluorescent Probes, Biophysical J. 82 2775-2783 (2002), ([41]), R. J. Ober, S. Ram, and E. S. Ward, Localization Accuracy in Single-Molecule Microscopy Biophysical J. 86, 1185-1200 (2004) ([42])).

L'analyse de l'interaction en fonction du déplacement observé et du flux appliqué peut être réalisée par tout moyen connu de l'homme du métier.

L'analyse de l'interaction peut être qualitative ou quantitative. Elle peut par exemple permettre de mesurer l'affinité entre la première et la deuxième molécule. Elle peut par exemple permettre de mesurer la constante d'association et/ou de dissociation de la première et de la deuxième molécule.

Par exemple, la mesure de la constante de dissociation entre la première et la deuxième molécule sous application d'une force F, $k_{off}(F)$, peut être obtenue quantitativement en mesurant le temps de détachement, sur un ensemble significatif de molécules - par exemple 30. Avantageusement, cette mesure donne accès au pourcentage de particules détachées en fonction du temps, qui suit une loi exponentielle de temps caractéristique $1/k_{off}(F)$. Ainsi, $k_{off}(F)$ peut être mesuré pour deux (ou plusieurs valeurs) de la force F.

La constante de dissociation en l'absence de force externe, $k_{off}(0)$, peut être déterminée par le modèle de Kramers (Kramers, H.A.," Brownian motion in a field of force and the diffusion model of chemical reactions" Physica (Amsterdam) 7, 284 (1940) ([43])) : $k_{off}(0) = w \exp(-\Delta E/k_B T)$, où w est un préfacteur, $\Delta E$ la barrière d'énergie à franchir pour la dissociation, $k_B$ la constante de Boltzmann et T la température de l'expérience. Lorsqu'on applique une force F, la barrière d'énergie est diminuée d'une énergie F.a, où a est la distance caractéristique du puits d'énergie (qui n'a pas besoin d'être connue). La nouvelle constante de dissociation est alors (Bell, G.I., Models for the specific adhesion of cells to cells. Science 200, 618-627 (1978) ([44])) :

$$k_{off}(F) = w \exp(-\Delta E/k_B T) \exp(Fa/k_B T) = k_{off}(0) \exp(Fa/k_B T).$$

Ainsi, à partir des constantes de dissociation obtenues expérimentalement pour au moins deux forces, $F_1$ et $F_2$, on peut déterminer la constante de dissociation en l'absence de force, $k_{off}(0)$, sans connaître la distance a. Des variantes de cette approche ont été proposées et peuvent également être utilisées (Evan Evans and Ken Ritchie, Dynamic Strength of Molecular Adhesion Bonds, Biophys. J. 72 1541-1555 (1997) ([45])).

Par exemple, la mesure de la constante d'équilibre entre la première et la deuxième molécule peut être faite en injectant une quantité connue de deuxième molécule attachée à la particule, en attendant un temps long devant à la fois celui d'attachement et celui de détachement. En rinçant à faible vitesse avec une solution sans deuxième molécule et sans nanoparticule, et en déterminant la fraction de particules restant attachées, on accède à la concentration attachée à l'équilibre, donc à celle détachée. Le rapport des concentrations attachée sur détachée donne la constante d'équilibre de la réaction. Si le temps d'attente est court devant le temps de détachement, la même méthode donne une mesure de la constante d'attachement : la quantité de particules attachées croit exponentiellement avec le temps.

Le procédé d'analyse de l'invention peut comprendre la détection des particules liées à une deuxième molécule n'ayant pas interagi avec la première molécule. Le dispositif peut, dans ce cas, comprendre un moyen de détection des particules ou des deuxièmes molécules en sortie du canal, c'est-à-dire à une extrémité à laquelle le flux arrive. Cette détection peut être basée sur les propriétés physiques (absorption, réflexion ou diffusion de la lumière, fluorescence, propriétés magnétiques ou électriques) et/ou chimiques (par exemple, interaction avec un autre réactant pour un dosage) des particules ou des deuxièmes molécules. Cette détection peut être avantageuse dans le cadre d'un procédé de screening de molécules lors desquels certaines deuxièmes molécules se lient aux premières molécules, tandis que d'autres ne se lient pas.

Un autre objet de l'invention est l'utilisation du procédé ou du dispositif dans un criblage d'une molécule candidate pour le développement d'un médicament.

Le procédé ou le dispositif de l'invention peut en outre être utilisé pour des mesures d'affinité en parallèle et à haut débit, de molécules entre elles.

Le procédé ou le dispositif de l'invention peut en outre être utilisé dans la recherche d'interactions entre cellules ou entre une cellule et une molécule.

Le procédé ou le dispositif de l'invention peut être utilisé dans l'évaluation de la mobilité de particules liées, soit entre elles, soit sur un support.

Le procédé ou le dispositif de l'invention peut être utilisé dans l'évaluation du mode de déplacement de particules liées.

Le procédé ou le dispositif de l'invention peut être utilisé dans l'évaluation de l'étirement, de l'élasticité et de repliement d'un polymère, d'une molécule d'acide nucléique, d'une protéine.

Le procédé ou le dispositif de l'invention peut en outre être utilisé dans l'évaluation des propriétés d'une membrane cellulaire. Dans ce cas, la première molécule peut être une cellule ou la membrane cellulaire, et la deuxième molécule peut être une molécule membranaire ou protéine membranaire de la cellule.

D'autres avantages pourront encore apparaître à l'homme du métier à la lecture de l'exemple ci-dessous, illustré par les figures annexées, données à titre illustratif.

## Brève description des figures

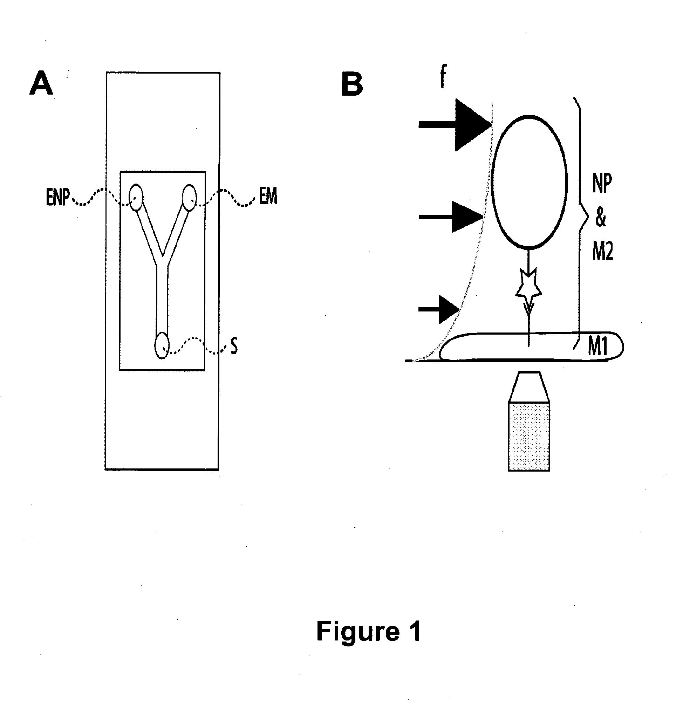

La figure 1A représente la géométrie du canal microfluidique, qui comprend une entrée de milieu de culture (EM), une entrée de solution de nanoparticules (ENP), une sortie (S) et une zone de mesure entre elles. La figure 1B illustre une méthode de mesure au moyen d'un microscope, dans laquelle un flux (f) est appliqué sur la nanoparticule (NP) et la deuxième molécule (M2), liée à la première molécule (M1). La deuxième molécule (M2) est constituée d'une toxine peptidique fixée sur son récepteur. La première molécule (M1) est constituée de la membrane cellulaire ainsi que de tous les éléments de la cellule (dont le cytosquelette) avec lesquels interagit le récepteur de la toxine. L'application du flux déterminé (f) sur la particule (NP) crée une force hydrodynamique sur la particule (NP) ; cette force est transmise à la deuxième molécule (M2) et permet d'observer l'effet de cette force sur l'interaction entre la première molécule (M1) et la deuxième molécule (M2).

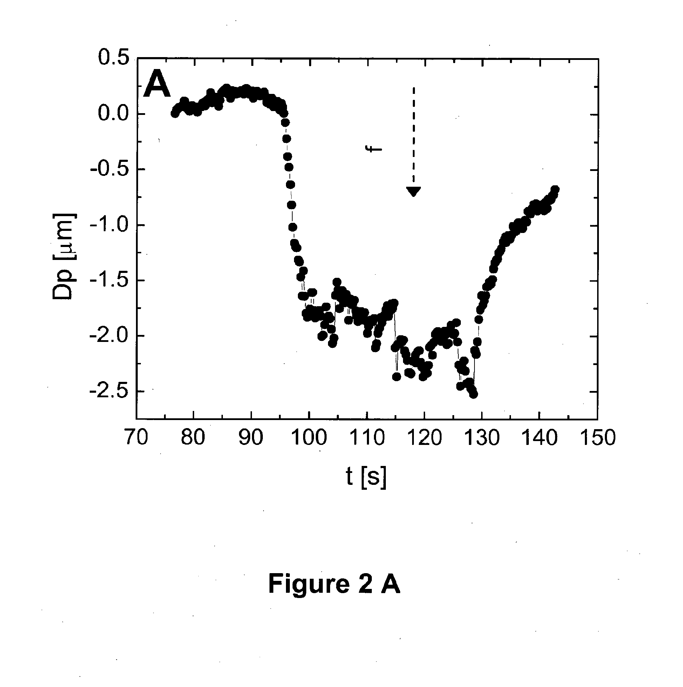

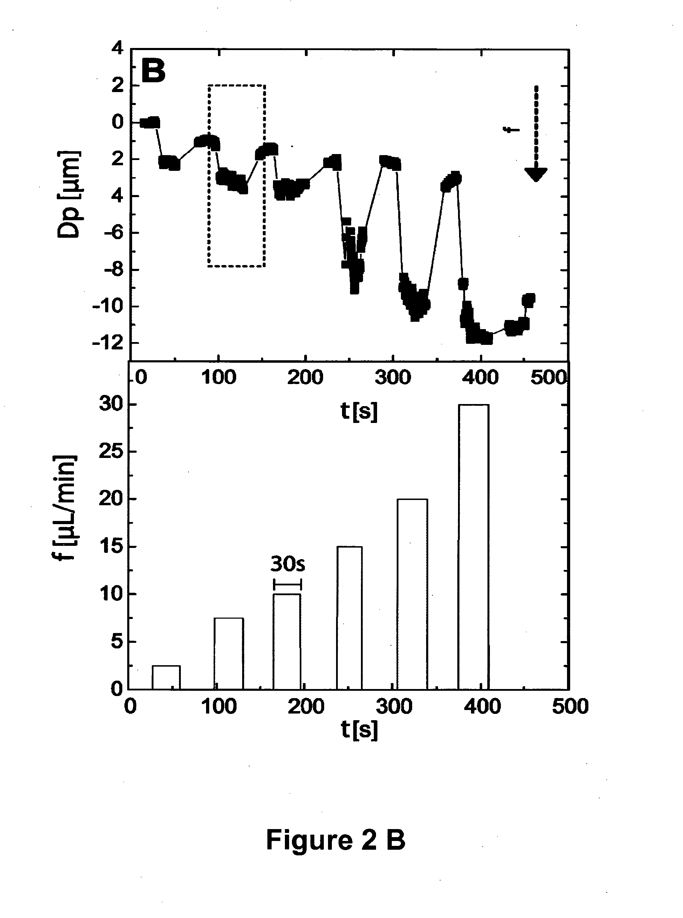

La figure 2A représente le déplacement (Dp) en $\mu$m du récepteur de la protéine toxine à la surface de la membrane cellulaire en fonction du temps (t, en secondes) suite à l'application d'un flux (f) de débit de 7,5 $\mu$L/min entre t=95 et 128s. La position du récepteur est visualisée à l'aide de la fluorescence des nanoparticules. Le récepteur se déplace dans la direction du flux (f) puis se stabilise à une nouvelle position décalée d'environ 2 $\mu$m par rapport à sa position initiale avant application du flux (f). Le déplacement dans la direction perpendiculaire à la direction du flux (f) est négligeable par rapport au déplacement dans la direction du flux (f). Après l'arrêt du flux (f), le récepteur revient à une position proche de la position initiale. Ce retour à une position proche de la position initiale laisse supposer que le récepteur est accroché au cytosquelette de la cellule. La position d'équilibre sous flux (f) correspondrait alors à la position pour laquelle la force appliquée sur le récepteur via la force hydrodynamique sur la particule est égale à la force de rappel de la cellule. A l'arrêt du flux (f), seule la force de rappel de la cellule agit sur le récepteur qui revient vers la position initiale avec une vitesse v, déterminée par la loi de Stokes : $F=\gamma v$, où $\gamma$ le coefficient de trainée du récepteur dans le système constitué par la membrane et le cytosquelette et F la force due au flux (f). Connaissant v et F, il est ainsi possible de déterminer le coefficient de frottement. La figure 2B (haut) représente le déplacement (Dp, en $\mu$m) en fonction du temps (t, en secondes) du récepteur par rapport à sa position initiale suite à l'application d'une succession de flux (f, en $\mu$L/min) de débits différents (bas). Les positions d'équilibre avec et sans flux sont montrées. Le rectangle tireté indique la partie de l'expérience qui est montrée dans la figure 2A.

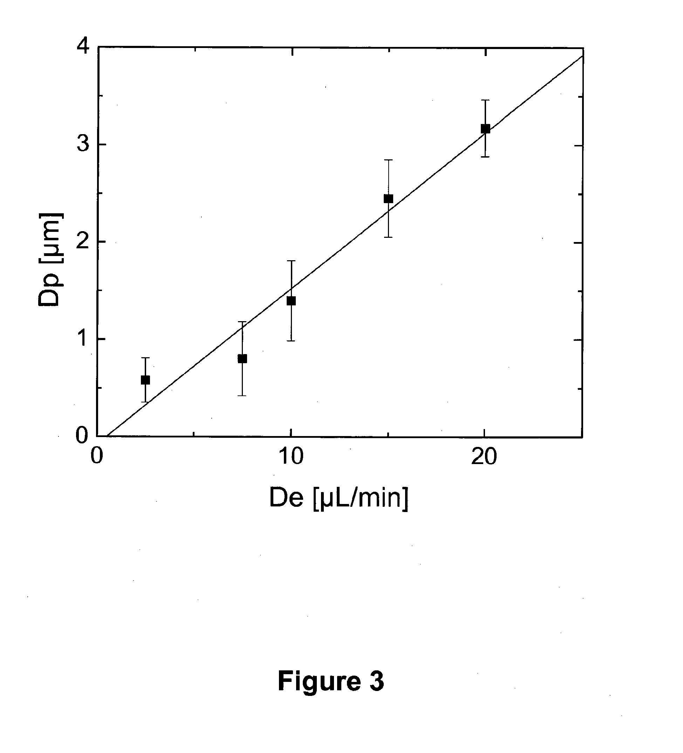

La figure 3 représente le déplacement moyen (Dp, en $\mu$m) en fonction de la force appliquée à l'aide du flux (f), dont le débit (De) est exprimé en $\mu$L/min, obtenu à partir d'expériences réalisées dans les mêmes conditions que celles de la figure 2B avec une séquence de débits (De) différents. Le déplacement du récepteur de la toxine dépend linéairement de la force appliquée. Comme indiqué, la position d'équilibre sous flux correspond à la position pour laquelle la force appliquée sur le récepteur via la force hydrodynamique sur la particule est égale à la force de rappel due à la cellule. La force de rappel de la cellule peut être modélisée par la loi de Hooke $F=k.x$, où k est la constante de raideur et x le déplacement par rapport à la position d'équilibre. Cette modélisation par la loi de Hooke fait l'hypothèse que la déformation de la cellule est élastique et le déplacement du récepteur réversible. Le récepteur de la toxine revient effectivement à une position proche de la position initiale. L'ajustement linéaire des données expérimentales de la figure 3 permet de déterminer la constante de raideur k.

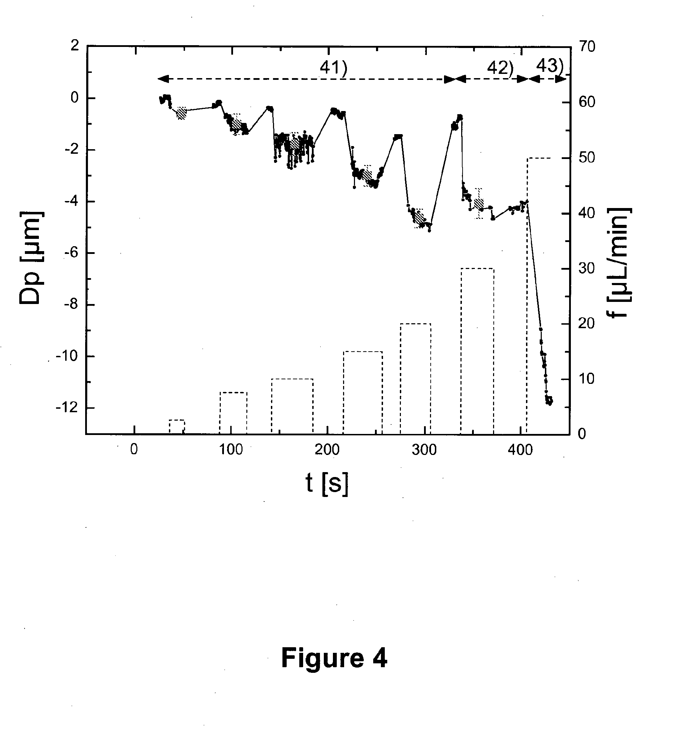

La figure 4 représente le déplacement (Dp, en $\mu$m) du récepteur en fonction du temps (t, en secondes) par rapport à sa position initiale suite à l'application d'une succession de flux (f, en $\mu$L/min) de débits différents (indiqués en tireté). Seules les positions d'équilibre avec et sans flux sont montrées dans cette figure. Plusieurs effets biomécaniques peuvent être identifiés pendant cette expérience. On observe un régime de déformation élastique, où le récepteur revient à sa position initiale après l'arrêt du flux (f). Après application d'un flux (f) de débit 30 $\mu$L/min, le récepteur ne revient pas à sa position initiale après l'arrêt du flux (f) : on se situe dans un régime de déformation plastique. Enfin, l'application d'un flux (f) de débit 50 $\mu$L/min, provoque le détachement de la toxine de son récepteur. L'observation d'un certain nombre d'événements de détachement peut être utilisée pour déterminer la constante de dissociation $k_{off}$ entre la toxine et son récepteur, comme expliqué dans la description de l'invention.

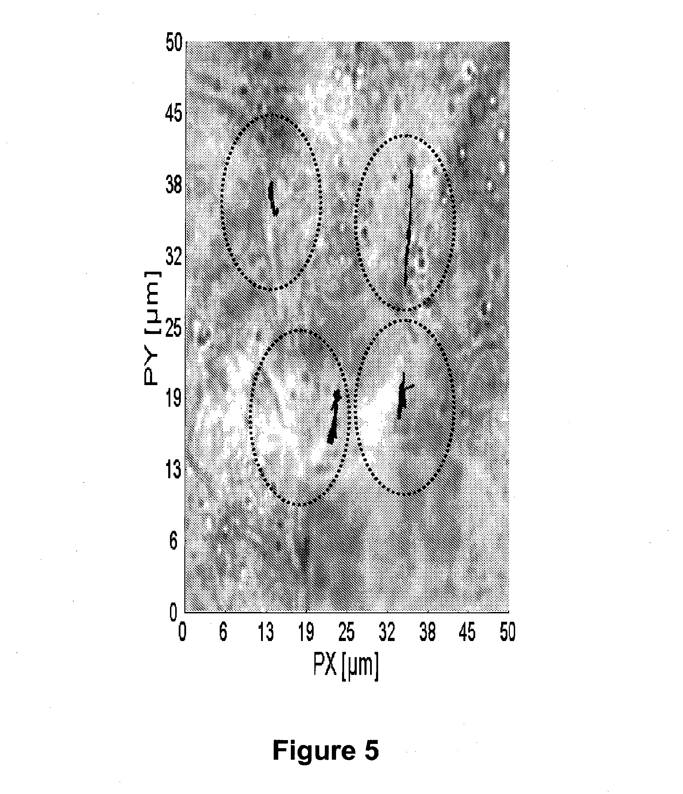

La figure 5 représente une image de cellules MDCK (Madin-Darby canine kidney) confluentes obtenue par transmission de lumière blanche. Les trajectoires de 4 récepteurs obtenues, représentées par la position X (PX en $\mu$m) et la position Y (PY en $\mu$m) suite à l'application d'une succession de flux de débits différents, comme sur la figure 2B, sont indiquées en traits noirs. Cette figure illustre la capacité de multiplexage de cette technique.

## EXEMPLES

## Exemple 1: ETUDE DES PROPRIÉTÉS DE LA MEMBRANE CELLULAIRE

### 1) Caractéristiques des particules et couplage à une toxine peptidique

Des nanoparticules (NPs) de vanadate d'Yttrium dopé par des ions Europium, $Y_{0.6}Eu_{0.4}VO_4$, de taille comprise entre 20 et 80 nm, sont obtenues par coprécipitation de $Y(NO_3)_3$, $Eu(NO_3)_3$ et $Na_3VO_4$, comme décrit dans le document Huignard et al. ([13]). Ces nanoparticules sont fluorescentes : les ions Europium à l'intérieur de ces nanoparticules peuvent être excités à 466 nm et émettent une fluorescence centrée à 617 nm.

La particule est couplée à une toxine peptidique par un cross-linker homobifonctionel, Bis(sulfosuccinimidyl)suberate ($BS_3$). Le méthode de couplage est la suivante et est décrite plus en détail par Casanova et al. (D. Casanova et al., J. Am. Chem. Soc. 129, 12592 (2007) ([46])) :

i) sélection des nanoparticules en taille par centrifugation
ii) transfert des NPs du solvant aqueux au solvant diméthylsulfoxyde (DMSO)
iii) première réaction d'acylation entre les nanoparticules et le cross-linker $BS_3$
iv) transfert des NPs du DMSO au solvant aqueux et seconde réaction entre nanoparticules-$BS_3$ et protéines
v) une séparation par centrifugation entre protéines libres et nanoparticules couplées aux protéines

La protéine choisie est la toxine epsilon produite par la bactérie anaérobie *Clostridium perfringens* types B et D. La toxine se fixe sur un récepteur spécifique qui est localisé dans des domaines résistants aux détergents dans les membranes des cellules MDCK (S. Miyata, J. Minami, E. Tamai, O. Matsushita, S. Shimamoto, A. Okabe, J. Biol. Chem. 277, 39463 (2002) ([47]), C. Chassin et al., Am. J. Pathol. Renal Physiol. 293, F927(2007) ([48]).

Lorsque l'on souhaite fixer une seule molécule sur la nanoparticule et lorsque la réaction de l'étape iv) a une efficacité proche de 100%, il faut choisir un rapport des concentrations des nanoparticules et des molécules d'intérêt, ici la toxine epsilon, proche de 1:1 pour la réalisation de l'étape iv). La concentration des nanoparticules couplées au $BS_3$ et des biomolécules avant réaction peut être déterminée par leur absorption. Après réaction, comme l'absorption des biomolécules et des nanoparticules se superpose, la concentration des protéines peut être déterminée par des tests standards tel que le test de Bradford.

Si l'efficacité de la réaction de l'étape iv) s'avère plus faible que 100%, le rapport des concentrations des nanoparticules et des molécules d'intérêt peut être ajusté en fonction. Le nombre de molécules liées à la nanoparticule suivra une distribution de Poisson. Il existera ainsi des nanoparticules sans molécule liée ainsi que des nanoparticules liées à une ou deux molécules ainsi qu'un faible nombre de nanoparticules liées à plus que deux molécules.

Les nanoparticules sans molécule liée ne vont pas interagir avec les récepteurs membranaires et ne vont pas perturber la mesure. Dans le cas des nanoparticules liées à deux ou trois molécules et étant donnée la taille des nanoparticules (20-80 nm), la probabilité que ces molécules soit disposées de façon à pouvoir interagir avec plus d'un récepteur est négligeable.

2) Caractéristiques du microcanal

Un microcanal est préparé en polydiméthylsiloxane (PDMS) par lithographie douce ([15]).

Les dimensions du microcanal sont de 50 $\mu$m en hauteur, de 500 $\mu$m en largeur et de 1,5 cm en longueur. Le microcanal a une géométrie en Y, et possède deux entrées : l'une pour l'injection des nanoparticules couplées à la toxine peptidique, et l'autre pour la génération du flux liquide déterminé.

Le microcanal possède également une sortie, connectée à un réservoir. Le réservoir permet de modifier la direction du flux sans risque d'introduire des bulles dans le microcanal.

3) Injection des cellules dans le microcanal

Une solution concentrée des cellules MDCK (~6x10$^6$ cellules par mL) dans leur milieu de culture (DMEM+10 % sérum de veau foetal (SVF) + 1 % pénicilline-streptomycine) est injectée dans le microcanal. Les cellules sont ensuite incubées à 37° et 5% $CO_2$ pendant une journée, de manière à ce que les cellules s'attachent à la surface du microcanal et s'y divisent.

La formation de bulles d'air doit être évitée, car des bulles passant à travers le microcanal risqueraient de détacher les cellules ou de dégrader la surface. Pour ce faire, le microcanal est complètement rempli avec du milieu de culture. Le remplissage du microcanal avec le milieu de culture un jour avant l'injection des cellules permet également d'enlever l'air présent dans le microcanal.

4) Injection des toxines peptidiques couplées aux nanoparticules dans le microcanal

Les nanoparticules couplées à la toxine peptidique sont suspendues avec une concentration de 0,05 mM en ions vanadate dans une solution de milieu minimal (HBSS+10mM HEPES+1% SVF). L'injection des nanoparticules est faite sur un microscope à l'aide d'un pousse-seringue. Une incubation est réalisée pendant 5-30 minutes afin que les toxines couplées aux particules se lient aux cellules, durée pendant laquelle aucun flux n'est appliqué.

Il est nécessaire à ce stade de vérifier que ces manipulations n'ont pas dégradé les cellules et qu'elles sont encore fixées sur la surface du microcanal par des images en transmission de lumière blanche et par l'observation du signal de fluorescence des cellules. Il est également utile de vérifier que les particules ont bien atteint la région du microcanal au niveau de laquelle sont fixées les cellules par microscopie de fluorescence.

5) Application du flux dans le microcanal et détermination de la force appliquée

La manipulation de l'échantillon avec le flux est faite sur un microscope. Le flux déterminé est appliqué dans le microcanal. Le flux déterminé est produit par l'injection, à l'aide d'un pousse-seringue, d'un volume de fluide choisi par unité de temps. Le fluide utilisé est du milieu minimal (HBSS+10mM HEPES+1% SVF). Cette méthode d'injection de fluide crée un flux de type Poiseuille dans le microcanal. L'écoulement du fluide est partout parallèle aux parois, la vitesse du fluide est nulle aux parois et la pression ne varie pas dans l'épaisseur de l'écoulement.

Pendant l'expérience, le flux est varié entre 0 et 200 $\mu$L/min. La force de trainée qui agit sur la particule est déterminée par la relation de Stokes :

$$F = 6\pi\mu Rv$$

où $\mu$ représente la viscosité du fluide, R le rayon hydrodynamique de la particule et v la vitesse du fluide autour de la particule. Cette relation est une approximation pour les canaux de longueur infinie. Comme la particule est située à une distance de la cellule comparable à son rayon hydrodynamique, un facteur correctif doit être appliqué à la formule ci-dessus ([16], [17], [18]), [19].

La viscosité du fluide est déterminée en mesurant le temps de son écoulement à travers un capillaire aux bouts duquel on applique une différence de pression ([14]). Nous avons mesuré une viscosité de 10$^{-3}$ Ns/m$^2$ à 20 °C. Le rayon de chaque particule est déterminé par le nombre de photons émis par unité de temps en employant la méthode décrite dans Casanova et al. ([46]). Pour des particules sphériques, le rayon hydrodynamique est très proche de la valeur ainsi déterminée.

Pour un débit de 10 $\mu$L/min, la vitesse moyenne du fluide est de 7 mm/s. Selon la loi de Poiseuille, la vitesse du fluide a un profil parabolique avec un maximum au centre du canal. Pour un débit de 10 $\mu$L/min, la vitesse maximale au centre du canal est de 10 mm/s. Ces vitesses n'endommagent pas le microcanal et n'induisent pas de détachement des cellules de la surface du microcanal. Des expériences de contrôle ont de plus permis de déterminer que ces débits n'occasionnent pas de déplacement des cellules ni de modification de la distribution des microtubules à l'intérieur de la cellule ni de modification de la distribution des radeaux lipidiques dans la membrane cellulaire.

La vitesse du fluide autour des particules a été déterminée par la technique de suivi des objets flottant dans le flux. Ici le suivi de nanoparticules fluorescentes $Y_{0,6}Eu_{0,4}VO_4$ a été utilisé.

Pour cette étude, le flux est appliqué dans une direction unique, à des débits variant de 0 à 200 μL/min. La valeur du débit est augmentée par palier pour mesurer le déplacement de la particule pour différentes valeurs du débit et donc de la force. Chaque valeur du débit est appliquée pendant environ 30 secondes, ensuite le flux est arrêté pendant environ 30 secondes, puis la nouvelle valeur de débit est appliquée (cf. Figure 2B (bas)).

Dans le cas de nos nanoparticules, la force appliquée peut varier entre 0 et quelques pN (piconewtons) par récepteur.

6) Enregistrement des images de fluorescence émise par les particules

Le suivi des particules est réalisé par le suivi du signal fluorescent émis par les particules par microscopie de fluorescence en champ large. Les nanoparticules sont excitées par un laser Argon à 465,8 nm à l'aide d'un miroir dichroïque 530DCXR (Chroma). Le laser est focalisé par un objectif de microscope (63x, NA = 1.4 oil immersion objective (Zeiss)). La largeur à mi-hauteur de la zone d'excitation est de 82 μm. La fluorescence émise par les particules est collectée par le même objectif et filtré par un filtre passe bande 617/8M (Chroma). Les images de fluorescence sont enregistrées par une caméra EM-CCD (Roper Scientific QuantEM:512SC). Le temps d'acquisition par image est de 200 ms.

7) Analyse des images et suivi du déplacement de la particule

Les images sont filtrées avec un filtre passe-bas et un filtre Laplace, pour enlever le bruit de fond. Après une étape de seuillage, la position des particules est déterminée par l'ajustement avec une Gaussienne 2D, ce qui permet de fournir le centre de la tâche d'émission de la particule avec une précision supérieure à la limite imposée par la résolution du microscope. Les positions successives de la particule obtenues à partir d'images enregistrées successivement permettent de mesurer son déplacement. Suite à l'application d'un débit constant, la particule se déplace et atteint une nouvelle position d'équilibre après quelques secondes. Le déplacement de la particule dans la figure 2A et 2B (haut) est indiqué par rapport à sa position initiale avant application du flux. Pour obtenir le déplacement moyen, la valeur moyenne de toutes les positions mesurées à l'équilibre après application d'un débit donné est calculée. Nous appelons la différence entre cette valeur moyenne et la position initiale avant application du flux le déplacement moyen. Ce déplacement moyen est montré sur la figure 3 en fonction de la force appliquée calculée pour différentes valeurs de débit.

**Listes des références**

1. Piehler et al., « New methodologies for measuring protein interactions in vivo and in vitro », Curr Opin Struct Biol. 2005 Feb;15(1):4-14.
2. Cooper MA, « Advances in membrane receptor screening and analysis », J Mol Recognit. 2004 Jul-Aug;17(4):286-315.
3. S. Chu, Laser Manipulation of Atoms and Particles, Science 253, 861 (1991).
4. A. Pralle, P. Keller, E.-L. Florin, K. Simons, and J.K.H. Hörber, Sphingolipid-Cholesterol Rafts Diffuse as Small Entities in the Plasma Membrane of Mammalian Cells, J. Cell Biol. 148, 997-1007 (2000).
5. Kenichi Suzuki, Ronald E. Sterba, and Michael P. Sheetz, Outer Membrane Monolayer Domains from Two-Dimensional Surface Scanning Resistance Measurements, Biophys. J. 79 448-459 (2000).
6. Moy, V. T., Florin, E.-L. & Gaub, H. E. Intermolecular forces and energies between ligands and receptors. Science 264, 257±259 (1994).
7. Hinterdorfer, P., Baumgartner, W., Gruber, H. J., Schilcher, K. & Schindler, H. Detection and localization of individual antibody-antigen recognition events by atomic force microscopy. Proc. Natl Acad. Sci. USA 93, 3477-3481 (1996).
8. Evans, E., Ritchie, K. & Merkel, R. Sensitive force technique to probe molecular adhesion and structural linkages at biological interfaces. Biophys. J. 68, 2580-2587 (1995).
9. R. Merkel, P. Nassoy, A. Leung, K. Ritchie, E. Evans, Nature 397, 50-53 (1999).
10. WO 2009098272.
11. WO 2006009398.
12. I. Barbulovic-Nad, M. Lucente, Y. Sun, M. Zhang, A. R. Wheeler, M. Bussmann, Bio-Microarray Fabrication Techniques-A Review, Critical Reviews in Biotechnology, 26:237-259, 2006.

13. Huignard, A.; Gacoin, T.; Boilot, J.-P. Chem. Mat. 2000, 12, 1090.

14. P. Cousson C. Ancey, Rhéophysique des pâtes et des suspensions, Ed. EDP Sciences (1999).

15. P. Tabelling, Introduction à la microfluidique, Editions Belin (2003), p. 178-202.

16. A. J. Goldman, R. G. Cox, H. Brenner, Slow viscous motion of a sphere parallel to a plane wall-I Motion through a quiescent fluid, Chem. Eng. Sci. 22 637-651 (1967).

17. A. J. Goldman, R. G. Cox, H. Brenner, Slow viscous motion of a sphere parallel to a plane wall-II Couette flow Chem. Eng. Sci. 22 653-660 (1967).

18. E. Lauga, T. M. Squires, Brownian motion near a partial-slip boundary: A local probe of the no-slip condition, Phys. Fluids 17, 103102 (2005).

19. J. Happel and H. Brenner, Low Reynolds Number Hydrodynamics, Martinus Nijhoff, The Hague (1983), p. 286-340.

20. Jin-Woo Choi, Kwang W. Oh, Jennifer H. Thomas, William R. Heineman, H. Brian, Halsall, Joseph H. Nevin, Arthur J. Helmicki H. Thurman Henderson and Chong H. Ahn, An integrated microfluidic biochemical détection system for protein analysis with magnetic bead-based sampling capabilities, Lab Chip, 2002, 2, 27-30.

21. Maria A. Schwarz and Peter C. Hauser, Recent developments in détection methods for microfabricated analytical devices, Lab on a Chip, 2001, 1, 1-6.

22. Jun Yang, Jianlong Zhao, Mengsu Yang, Optical and electrochemical détection techniques for cell-based microfluidic systems, Anal Bioanal Chem (2006) 384: 1259-1268.

23. Homola J, "Present and future of surface plasmon resonance biosensors", ANALYTICAL AND BIOANALYTICAL CHEMISTRY 377, 528-53 (2003).

24. Molloy JE, Padgett MJ, "Lights, action: optical tweezers", CONTEMP. PHYS. 43, 241-258 (2002).

25. D. Boyer, P. Tamarat, A. Maali, B. Lounis, M. Orrit, "Photothermal Imaging of Nanometer-Sized Metal Particles Among Scatterers", Science 297, 1160 (2002).

26. D. Lasne, G. A. Blab, S. Berciaud, M. Heine, L. Groc, D. Choquet, L. Cognet, B. Lounis, "Single Nanoparticle Photothermal Tracking (SNaPT) of 5-nm Gold Beads in Live Cells", Biophys. J. 91, 4598-4604 (2006).

27. Arbouet A, Christofilos D, Del Fatti N, Vallee F, Huntzinger JR, Arnaud L, Billaud P, Broyer M, "Direct measurement of the single-metal-cluster optical absorption", Phys. Rev. Lett. 93, 127401 (2004).

28. Muskens OL, Del Fatti N, Vallee F, Huntzinger JR, Billaud P, Broyer M, "Single metal nanoparticle absorption spectroscopy and optical characterization", Appl. Phys. Lett. 88, 063109 (2006).

29. De Brabander, M., R. Nuydens, G. Geuens, M. Moeremans, and J. de Mey, "The use of submicroscopic gold particles combined with video contrast enhancement as a simple molecular probe for the living cell". Cell Motil. Cytoskeleton. 6:105-113 (1986).

30. D. Boyer, P. Tamarat, A. Maali, B. Lounis, M. Orrit, "Photothermal Imaging of Nanometer-Sized Metal Particles Among Scatterers", Science 297, 1160 (2002).

31. D. Lasne, G. A. Blab, S. Berciaud, M. Heine, L. Groc, D. Choquet, L. Cognet, B. Lounis, "Single Nanoparticle Photothermal Tracking (SNaPT) of 5-nm Gold Beads in Live Cells", Biophys. J. 91, 4598-4604 (2006).

32. Homola J, "Present and future of surface plasmon resonance biosensors". Anal Bioanal Chem, vol. 377, 528-539, 2003.

33. Chen et al. Nano Letters, vol. 7, No. 3, 690-696, 2007.

34. Bek et al., Nano Letters, vol. 8, No. 2, 485-490, 2008.

35. WO2007036544.

36. Aslan et al., Current Opinion in Chemical Biology, 9 :538-544, 2005.

37. W. R. Zipfel, R. M. Williams, W. W. Webb, Nonlinear magic: multiphoton microscopy in the biosciences, Nat. Biotech. 21, 1369 (2003).

38. A. L. Stout and D. Axelrod, Evanescent field excitation of fluorescence by epi-illumination microscopy, Appl. Opt. 28, 5237 (1989).

39. N. L. Thompson and B. C. Lagerholm, Total internal reflection fluorescence: applications in cellular biophysics, Curr. Opin. Biotech., 8, 58-64 (1997).

40. M. K. Cheezum, W. F. Walker, and W. H. Guilford, Quantitative Comparison of Algorithms for Tracking Single Fluorescent Particles, Biophys. J. 81 2378-2388 (2001).

41. R. E. Thompson, D. R. Larson, and W. W. Webb, Precise Nanometer Localization Analysis for Individual Fluorescent Probes, Biophysical J. 82 2775-2783 (2002).

42. R. J. Ober, S. Ram, and E. S. Ward, Localization Accuracy in Single-Molecule Microscopy Biophysical J. 86, 1185-1200 (2004).

43. Kramers, H.A.," Brownian motion in a field of force and the diffusion model of chemical reactions" Physica (Amsterdam) 7, 284 (1940).

44. Bell, G.I.,. Models for the specific adhesion of cells to cells. Science 200, 618-627 (1978).

45. Evan Evans and Ken Ritchie, Dynamic Strength of Molecular Adhesion Bonds, Biophys. J. 72 1541-1555 (1997).

46. Casanova et al., Appl. Phys. Lett. 89, 253103 (2006).

47. S. Miyata, J. Minami, E. Tamai, O. Matsushita, S. Shimamoto, A. Okabe, J. Biol. Chem. 277, 39463 (2002).

48. C. Chassin et al., Am. J. Pathol. Renal Physiol. 293, F927 (2007).

49. Schmidt Brian J. et al. "Catch Strip Assay for the Relative Assessment of Two-Dimensional Protein Association Kinetics", Anal. Chem. 2008, 80 :944-950

**Revendications**

1. Procédé d'analyse d'une interaction entre une première molécule et une deuxième molécule liée à une particule, comprenant les étapes suivantes :

   - mettre en contact la première molécule et la deuxième molécule liée à la particule dans des conditions rendant possible leur interaction,
   - appliquer un flux liquide déterminé sur la particule liée à la deuxième molécule,
   - observer un déplacement de la particule liée à la deuxième molécule sous l'action du flux appliqué,
   - analyser l'interaction en fonction du déplacement observé et du flux appliqué,

   la particule ayant une résistance hydrodynamique supérieure à celle de la première et/ou de la deuxième molécule, un nombre de Péclet massique supérieur à 1, et une dimension de taille nanométrique comprise entre 1 et 100 nm.

2. Procédé selon la revendication 1, dans lequel le déplacement de la particule est observé grâce aux propriétés physiques et/ou chimiques de la particule.

3. Procédé selon l'une quelconque des revendications 1 ou 2, dans lequel l'observation est réalisée par une méthode choisie dans le groupe comprenant une méthode optique, une méthode chimique, une méthode électrochimique et une méthode magnétique.

4. Procédé selon la revendication 3, dans lequel l'observation est réalisée par une méthode optique choisie dans le groupe comprenant l'utilisation d'un émetteur de fluorescence et d'un moyen de détection de la fluorescence, une détection de l'absorption, une détection de la réflexion, une détection de la diffusion et une détection de la diffraction.

5. Procédé selon la revendication 4, dans lequel l'émetteur de fluorescence est la particule elle-même ou un marqueur associé à la particule et/ou à au moins une molécule choisie parmi la première molécule et la deuxième molécule.

6. Procédé selon l'une quelconque des revendications précédentes, dans lequel le flux liquide déterminé est tel qu'il permet d'appliquer à la particule une force F égale à $6\pi\mu Rv$, dans laquelle $\mu$ représente la viscosité du fluide, R le rayon hydrodynamique de la particule et v la vitesse du fluide autour de la particule.

7. Procédé selon l'une quelconque des revendications précédentes, dans lequel l'analyse est qualitative ou quantitative.

8. Procédé selon l'une quelconque des revendications précédentes, dans lequel l'interaction est une interaction entre des molécules biologiques ou entre une molécule biologique et une molécule chimique.

9. Procédé selon l'une quelconque des revendications précédentes, dans lequel la première molécule est une cellule ou une membrane cellulaire.

10. Dispositif d'analyse d'une interaction entre une première molécule et au moins une deuxième molécule, comprenant: un moyen d'application d'un flux liquide déterminé, un support d'accueil d'une première molécule, une particule capable de lier une deuxième molécule susceptible d'interagir avec la première molécule, ladite particule ayant une dimension de taille nanométrique comprise entre 1 et 100 nm, un moyen d'observation du déplacement de la particule, et un moyen de contrôle du flux liquide déterminé, dans lequel la particule a une résistance hydrodynamique supérieure à celle de la première et/ou de la deuxième molécule et un nombre de Péclet massique supérieur à 1.

11. Dispositif selon la revendication 10, dans lequel le moyen d'application du flux liquide déterminé est un moyen permettant d'appliquer à la particule, via le flux liquide, une force F égale à $6\pi\mu Rv$, dans laquelle $\mu$ représente la viscosité du fluide, R le rayon de la particule et v la vitesse du fluide autour de la particule.

**12.** Dispositif selon l'une quelconque des revendications 10 ou 11, dans lequel le moyen d'observation du déplacement de la particule est un moyen d'observation utilisant des propriétés physiques et/ou chimiques de la particule.

**13.** Utilisation du procédé selon l'une quelconque des revendications 1 à 9 ou du dispositif selon l'une quelconque des revendications 10 à 12, dans un criblage d'une molécule candidate pour le développement d'un médicament.

**Patentansprüche**

**1.** Verfahren zur Analyse einer Wechselwirkung zwischen einem ersten Molekül und einem an ein Teilchen gebundenen zweiten Molekül, das die folgenden Schritte umfasst:

- Inberührungbringen des ersten Moleküls und des an das Teilchen gebundenen zweiten Moleküls unter Bedingungen, die ihre Wechselwirkung ermöglichen,
- Anwenden eines bestimmten flüssigen Stroms auf das an das zweite Molekül gebundene Teilchen,
- Beobachten einer Verschiebung des an das zweite Molekül gebundenen Teilchens unter der Einwirkung des angewendeten Stroms,
- Analysieren der Wechselwirkung in Abhängigkeit von der beobachteten Verschiebung und dem angewendeten Strom,

wobei das Teilchen einen größeren hydrodynamischen Widerstand als das erste und/oder das zweite Molekül, eine massenbezogene Peclet-Zahl von mehr als 1 und eine nanometrische Größenabmessung zwischen 1 und 100 nm aufweist.

**2.** Verfahren nach Anspruch 1, bei dem die Verschiebung des Teilchens mit Hilfe der physikalischen und/oder chemischen Eigenschaften des Teilchens beobachtet wird.

**3.** Verfahren nach Anspruch 1 oder 2, bei dem die Beobachtung durch eine Methode aus der Gruppe umfassend eine optische Methode, eine chemische Methode, eine elektrochemische Methode und eine magnetische Methode durchgeführt wird.

**4.** Verfahren nach Anspruch 3, bei dem die Beobachtung durch eine optische Methode aus der Gruppe umfassend die Verwendung eines Fluoreszenzemitters und eines Mittels zur Detektion von Fluoreszenz, eine Detektion von Absorption, eine Detektion von Reflexion, eine Detektion von Diffusion und eine Detektion von Diffraktion durchgeführt wird.

**5.** Verfahren nach Anspruch 4, bei dem es sich bei dem Fluoreszenzemitter um das Teilchen selbst oder einen mit dem Teilchen und/oder mit mindestens einem aus dem ersten Molekül und dem zweiten Molekül ausgewählten Molekül assoziierten Marker handelt.

**6.** Verfahren nach einem der vorhergehenden Ansprüche, bei dem der bestimmte flüssige Strom derart beschaffen ist, dass er das Anwenden einer Kraft F gleich $6\pi\mu Rv$ auf das Teilchen ermöglicht, wobei $\mu$ für die Viskosität des Fluids steht, R für den hydrodynamischen Radius des Teilchens steht und v für die Geschwindigkeit des Fluids um das Teilchen herum steht.

**7.** Verfahren nach einem der vorhergehenden Ansprüche, bei dem die Analyse qualitativ oder quantitativ ist.

**8.** Verfahren nach einem der vorhergehenden Ansprüche, bei dem es sich bei der Wechselwirkung um eine Wechselwirkung zwischen biologischen Molekülen oder zwischen einem biologischen Molekül und einem chemischen Molekül handelt.

**9.** Verfahren nach einem der vorhergehenden Ansprüche, bei dem es sich bei dem ersten Molekül um eine Zelle oder eine Zellmembran handelt.

**10.** Vorrichtung zur Analyse einer Wechselwirkung zwischen einem ersten Molekül und mindestens einem zweiten Molekül, umfassend: ein Mittel zum Anwenden eines bestimmten flüssigen Stroms, einen Träger zum Aufnehmen eines ersten Moleküls, ein Teilchen, das an ein zweites Molekül, das mit dem ersten Molekül wechselwirken kann, binden kann, wobei das Teilchen eine nanometrische Größenabmessung zwischen 1 und 100 nm aufweist, ein Mittel zum Beobachten der Verschiebung des Teilchens und ein Mittel zur Steuerung des bestimmten flüssigen Stroms, wobei das Teilchen einen größeren hydrodynamischen Widerstand als das erste und/oder das zweite Molekül und eine massenbezogene Peclet-Zahl von mehr als 1 aufweist.

11. Vorrichtung nach Anspruch 10, wobei es sich bei dem Mittel zum Anwenden des bestimmten flüssigen Stroms um ein Mittel handelt, das das Anwenden einer Kraft F gleich $6\pi\mu Rv$ auf das Teilchen über den flüssigen Strom ermöglicht, wobei $\mu$ für die Viskosität des Fluids steht, R für den Radius des Teilchens steht und v für die Geschwindigkeit des Fluids um das Teilchen herum steht.

12. Vorrichtung nach Anspruch 10 oder 11, bei dem es sich bei dem Mittel zum Beobachten der Verschiebung des Teilchens um ein Mittel zum Beobachten unter Verwendung der physikalischen und/oder chemischen Eigenschaften des Teilchens handelt.

13. Verwendung des Verfahrens nach einem der Ansprüche 1 bis 9 oder der Vorrichtung nach einem der Ansprüche 10 bis 12 bei der Durchmusterung eines Kandidatenmoleküls für die Entwicklung eines Medikaments.

## Claims

1. A method for analyzing an interaction between a first molecule and a second molecule bonded to a particle, comprising the following steps:

   - bringing the first molecule and the second molecule bonded to the particle into contact under conditions enabling the interaction thereof,
   - applying a predetermined liquid flow to the particle bonded to the second molecule,
   - observing a moving of the particle bonded to the second molecule caused by the applied flow,
   - analyzing the interaction according to the moving observed and to the applied flow,

   the particle having a greater hydrodynamic resistance than the first and/or second molecule, a mass Péclet number greater than 1, and a nanometric size dimension between 1 and 100 nm.

2. The method according to claim 1, wherein the moving of the particle is observed by means of the physical and/or chemical properties of the particle.

3. The method according to any one of claims 1 or 2, wherein the observation is carried out by a method selected from the group comprising an optical method, a chemical method, an electrochemical method and a magnetic method.

4. The method according to claim 3, wherein the observation is carried out by an optical method selected from the group comprising the use of a fluorescence emitter and of a means for detecting fluorescence, detecting of absorption, detecting of reflection, detecting of scattering and detecting of diffraction.

5. The method according to claim 4, wherein the fluorescence emitter is the particle itself or a label associated with the particle and/or with at least one molecule selected from the first molecule and the second molecule.

6. The method according to any one of the preceding claims, wherein the predetermined liquid flow is such that it makes it possible to apply to the particle a force F equal to $6\pi\mu Rv$, wherein $\mu$ represents the viscosity of the fluid, R the hydrodynamic radius of the particle and v the speed of the fluid around the particle.

7. The method according to any one of the preceding claims, wherein the analysis is qualitative or quantitative.

8. The method according to any one of the preceding claims, wherein the interaction is an interaction between biological molecules or between a biological molecule and a chemical molecule.

9. The method according to any one of the preceding claims, wherein the first molecule is a cell or a cell membrane.

10. A device for analyzing an interaction between a first molecule and at least one second molecule, comprising: a means of applying a predetermined liquid flow, a support for receiving a first molecule, a particle capable of bonding a second molecule capable of interacting with the first molecule, said particle having a nanometric size dimension between 1 and 100 nm, a means for observing the moving of the particle, and a means of controlling the predetermined liquid flow, in which the particle has a greater hydrodynamic resistance than the first and/or second molecule, and a mass Péclet number of greater than 1.

11. The device according to claim 10, wherein the means for applying the predetermined liquid flow is a means which makes it possible to apply to the particle, via the liquid flow, a force F equal to $6\pi\mu Rv$, in which $\mu$ represents the viscosity of the fluid, R the radius of the particle and v the speed of the fluid around the particle.

12. The device according to any one of claims 10 or 11, wherein the means for observing the moving of the particle is a means of observation using physical and/or chemical properties of the particle.

13. The use of the method according to any one of claims 1 to 9 or of the device according to any one of claims 10 to 12, in screening for a candidate molecule for developing a medicament.

**Figure 1**

**Figure 2 A**

Figure 2 B

**Figure 3**

**Figure 4**

**Figure 5**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2009098272 A
- WO 2006009398 A
- WO 03095973 A
- WO 2007092909 A
- WO 2007036544 A

**Littérature non-brevet citée dans la description**

- **PIEHLER et al.** New methodologies for measuring protein interactions in vivo and in vitro. *Curr Opin Struct Biol.,* Février 2005, vol. 15 (1), 4-14
- **COOPER MA.** Advances in membrane receptor screening and analysis. *J Mol Recognit.,* Juillet 2004, vol. 17 (4), 286-315
- **S. CHU.** Laser Manipulation of Atoms and Particles. *Science,* 1991, vol. 253, 861
- **A. PRALLE ; P. KELLER ; E.-L. FLORIN ; K. SIMONS ; J.K.H. HÖRBER.** Sphingolipid-Cholesterol Rafts Diffuse as Small Entities in the Plasma Membrane of Mammalian Cells. *J. Cell Biol.,* 2000, vol. 148, 997-1007
- **KENICHI SUZUKI ; RONALD E. STERBA ; MICHAEL P. SHEETZ.** Outer Membrane Monolayer Domains from Two-Dimensional Surface Scanning Resistance Measurements. *Biophys. J.,* 2000, vol. 79, 448-459
- **MOY, V. T. ; FLORIN, E.-L. ; GAUB, H. E.** Intermolecular forces and energies between ligands and receptors. *Science,* 1994, vol. 264, 257-259
- **HINTERDORFER, P. ; BAUMGARTNER, W. ; GRUBER, H. J. ; SCHILCHER, K. ; SCHINDLER, H.** Detection and localization of individual antibody-antigen recognition events by atomic force microscopy. *Proc. Natl Acad. Sci. USA,* 1996, vol. 93, 3477-3481
- **EVANS, E. ; RITCHIE, K. ; MERKEL, R.** Sensitive force technique to probe molecular adhesion and structural linkages at biological interfaces. *Biophys. J.,* 1995, vol. 68, 2580-2587
- **R. MERKEL ; P. NASSOY ; A. LEUNG ; K. RITCHIE ; E. EVANS.** *Nature,* 1999, vol. 397, 50-53
- **MIALON G. et al.** Luminescent oxide nanoparticles with enhanced optical properties. *JOURNAL OF LUMINESCENCE, AMSTERDAM, NL,* Décembre 2009, vol. 129 (12), 1706-1710
- Inferring maps of forces inside cell membrane micro-domains. **MASSON J -B. et al.** PHYSICAL REVIEW LETTERS. AMERICAN PHYSICAL SOCIETY, Janvier 2009, vol. 102
- **BEAUREPAIRE E. et al.** Functionalized luminescent oxide nanoparticles for sodium channel imaging at the single molecule level. *PROCEEDINGS OF THE SPIE - THE INTERNATIONAL SOCIETY FOR OPTICAL ENGINEERING SPIE - USA,* Avril 2005, vol. 5705 (1), 192-198
- **SCHMIDT BRIAN J. et al.** Catch Strip Assay for the Relative Assessment of Two-Dimensional Protein Association Kinetics. *Anal. Chem.,* 2008, vol. 80, 944-950
- **SCHMIDT BRIAN J. et al.** *Anal. Chem.,* 2008, vol. 80, 944-950
- **I. BARBULOVIC-NAD ; M. LUCENTE ; Y. SUN ; M. ZHANG ; A. R. WHEELER ; M. BUSSMANN.** *Bio-Microarray Fabrication Techniques-A Review, Critical Reviews in Biotechnology,* 2006, vol. 26, 237-259
- **A. J. GOLDMAN ; R. G. COX ; H. BRENNER.** Slow viscous motion of a sphere parallel to a plane wall-I Motion through a quiescent fluid. *Chem. Eng. Sci.,* 1967, vol. 22, 637-651
- **A. J. GOLDMAN ; R. G. COX ; H. BRENNER.** Slow viscous motion of a sphere parallel to a plane wall-II Couette flow. *Chem. Eng. Sci.,* 1967, vol. 22, 653-660
- **E. LAUGA ; T. M. SQUIRES.** Brownian motion near a partial-slip boundary: A local probe of the no-slip condition. *Phys. Fluids,* 2005, vol. 17, 103102
- **J. HAPPEL ; H. BRENNER.** *Low Reynolds Number Hydrodynamics,* 1983, 286-340
- **HUIGNARD, A. ; GACOIN, T. ; BOILOT, J.** *P. Chem. Mat.,* 2000, vol. 12, 1090
- **P. COUSSON ; C. ANCEY.** Rhéophysique des pâtes et des suspensions. 1999
- **P. TABELLING.** Introduction à la microfluidique. 2003, 178-202

- **JIN-WOO CHOI ; KWANG W. OH ; JENNIFER H. THOMAS ; WILLIAM R. HEINEMAN ; H. BRIAN, HALSALL ; JOSEPH H. NEVIN ; ARTHUR J. ; HELMICKI H.** Thurman Henderson and Chong H. Ahn, An integrated microfluidic biochemical détection system for protein analysis with magnetic bead-based sampling capabilities. *Lab Chip,* 2002, vol. 2, 27-30
- **MARIA A. SCHWARZ ; PETER C. HAUSER.** Récent developments in détection methods for microfabricated analytical devices. *Lab on a Chip,* 2001, vol. 1, 1-6
- **JUN YANG ; JIANLONG ZHAO ; MENGSU YANG.** Optical and electrochemical détection techniques for cell-based microfluidic systems. *Anal Bioanal Chem,* 2006, vol. 384, 1259-1268
- **HOMOLA J.** Présent and future of surface plasmon résonance biosensors. *ANALYTICAL AND BIOANALYTICAL CHEMISTRY,* 2003, vol. 377, 528-53
- **MOLLOY JE ; PADGETT MJ.** Lights, action: optical tweezers. *CONTEMP. PHYS.,* 2002, vol. 43, 241-258
- **D. BOYER ; P. TAMARAT ; A. MAALI ; B. LOUNIS ; M. ORRIT.** Photothermal Imaging of Nanometer-Sized Metal Particles Among Scatterers. *Science,* 2002, vol. 297, 1160
- **D. LASNE ; G. A. BLAB ; S. BERCIAUD ; M. HEINE ; L. GROC ; D. CHOQUET ; L. COGNET ; B. LOUNIS.** Single Nanoparticle Photothermal Tracking (SNaPT) of 5-nm Gold Beads in Live Cells. *Biophys. J.,* 2006, vol. 91, 4598-4604
- **ARBOUET A ; CHRISTOFILOS D ; DEL FATTI N ; VALLEE F ; HUNTZINGER JR ; ARNAUD L ; BILLAUD P ; BROYER M.** Direct measurement of the single-metal-cluster optical absorption. *Phys. Rev. Lett.,* 2004, vol. 93, 127401
- **MUSKENS OL ; DEL FATTI N ; VALLEE F ; HUNTZINGER JR ; BILLAUD P ; BROYER M.** Single metal nanoparticle absorption spectroscopy and optical characterization. *Appl. Phys. Lett.,* 2006, vol. 88, 063109
- **DE BRABANDER ; M., R. NUYDENS ; G. GEUENS ; M. MOEREMANS ; J. DE MEY.** The use of submicroscopic gold particles combined with video contrast enhancement as a simple molecular probe for the living cell. *Cell Motil. Cytoskeleton.,* 1986, vol. 6, 105-113
- **M. J. SAXTON ; K. JACOBSON.** SINGLE-PARTICLE TRACKING:Applications to Membrane Dynamics. *Annu. Rev. Biophys. Biomol. Struct.,* 1997, vol. 26, 373-99
- **CHEN et al.** *Nano Letters,* 2007, vol. 7 (3), 690-696
- **BEK et al.** *Nano Letters,* 2008, vol. 8 (2), 485-490
- **ASLAN et al.** *Current Opinion in Chemical Biology,* 2005, vol. 9, 538-544
- **W. R. ZIPFEL ; R. M. WILLIAMS ; W. W.WEBB.** Nonlinear magic: multiphoton microscopy in the biosciences. *Nat. Biotech.,* 2003, vol. 21, 1369
- **A. L. STOUT ; D. AXELROD.** Evanescent field excitation of fluorescence by epi-illumination microscopy. *Appl. Opt.,* 1989, vol. 28, 5237
- **N. L. THOMPSON ; B. C. LAGERHOLM.** Total internal reflection fluorescence: applications in cellular biophysics. *Curr. Opin. Biotech.,* 1997, vol. 8, 58-64
- **M. K. CHEEZUM ; W. F. WALKER ; W. H. GUILFORD.** Quantitative Comparison of Algorithms for Tracking Single Fluorescent Particles. *Biophys. J.,* 2001, vol. 81, 2378-2388
- **R. E. THOMPSON ; D. R. LARSON ; W. W. WEBB.** Précise Nanometer Localization Analysis for Individual Fluorescent Probes. *Biophysical J.,* 2002, vol. 82, 2775-2783
- **R. J. OBER ; S. RAM ; E. S. WARD.** *Localization Accuracy in Single-Molecule Microscopy Biophysical J.,* 2004, vol. 86, 1185-1200
- **KRAMERS, H.A.** Brownian motion in a field of force and the diffusion model of chemical reactions. *Physica,* 1940, vol. 7, 284
- **BELL, G.I.** Models for the specific adhesion of cells to cells. *Science,* 1978, vol. 200, 618-627
- **EVAN EVANS ; KEN RITCHIE.** Dynamic Strength of Molecular Adhesion Bonds. *Biophys. J.,* 1997, vol. 72, 1541-1555
- **D. CASANOVA et al.** *J. Am. Chem. Soc.,* 2007, vol. 129, 12592
- **S. MIYATA ; J. MINAMI ; E. TAMAI ; O. MATSUSHITA ; S. SHIMAMOTO ; A. OKABE.** *J. Biol. Chem.,* 2002, vol. 277, 39463
- **C. CHASSIN et al.** *Am. J. Pathol. Renal Physiol.,* 2007, vol. 293, F927
- **I. BARBULOVIC-NAD ; M. LUCENTE ; Y. SUN ; M. ZHANG ; A. R. WHEELER ; M. BUSSMANN.** Bio-Microarray Fabrication Techniques-A Review. *Critical Reviews in Biotechnology,* 2006, vol. 26, 237-259
- Introduction à la microfluidique. **P. TABELLING.** Editions Belin. 2003, 178-202
- **A. J. GOLDMAN ; R. G. COX ; H. BRENNER.** Slow viscous motion of a sphere parallel to a plane wall-I Motion through a quiescent fluid. *Chem. Eng.,* 1967, vol. 22, 637-651
- **J. HAPPEL ; H. BRENNER.** Low Reynolds Number Hydrodynamics. *Martinus Nijhoff, The Hague,* 1983, 286-340

- **JIN-WOO CHOI ; KWANG W. OH ; JENNIFER H. THOMAS ; WILLIAM R. HEINEMAN ; H. BRIAN, HALSALL ; JOSEPH H. NEVIN ; ARTHUR J. ; HELMICKI H.** Thurman Henderson and Chong H. Ahn, An integrated microfluidic biochemical détection system for protein analysis with magnetic bead-based sampling capabilities. *Lab Chip,* 2002, vol. 2, 27-30
- **MARIA A. SCHWARZ ; PETER C. HAUSER.** Recent developments in détection methods for microfabricated analytical devices. *Lab on a Chip,* 2001, vol. 1, 1-6
- **HOMOLA J.** Present and future of surface plasmon resonance biosensors. *ANALYTICAL AND BIOAN-ALYTICAL CHEMISTRY,* 2003, vol. 377, 528-53

- **DE BRABANDER, M. ; R. NUYDENS ; G. GEUENS ; M. MOEREMANS ; J. DE MEY.** The use of submicroscopic gold particles combined with video contrast enhancement as a simple molecular probe for the living cell. *Cell Motil. Cytoskeleton.,* 1986, vol. 6, 105-113
- **HOMOLA J.** Present and future of surface plasmon resonance biosensors. *Anal Bioanal Chem,* 2003, vol. 377, 528-539
- **R. E. THOMPSON ; D. R. LARSON ; W. W. WEBB.** Precise Nanometer Localization Analysis for Individual Fluorescent Probes. *Biophysical J.,* 2002, vol. 82, 2775-2783
- **CASANOVA et al.** *Appl. Phys. Lett.,* 2006, vol. 89, 253103